# EUROPÄISCHE PATENTSCHRIFT

(11) **EP 2 557 161 B1**
(45) Veröffentlichungstag und Bekanntmachung des Hinweises auf die Patenterteilung: **20.05.2015**
(21) Anmeldenummer: 12171498.4
(22) Anmeldetag: 11.06.2012
(51) Int. Cl.: C12N 15/10

(54) **Verfahren zur Isolierung von RNA aus Vollblutproben**
Method for isolating RNA from full blood samples
Procédé destiné à l'isolation d'ARN à partir d'échantillons de sang total

(30) Priorität: 11.08.2011 DE 102011080853
(43) Veröffentlichungstag der Anmeldung: 13.02.2013
(73) Patentinhaber: AXAGARIUS GmbH & Co. KG, 52355 Düren (DE)
(72) Erfinder: Kirsch, Christoph, Dr., 50259 Pulheim (DE); Bayard, Claudia, 53909 Zülpich (DE); Meusel, Markus, Dr., 52146 Würselen (DE); Zinn, Thomas, Dr., 52355 Düren (DE); Wagner, Carolin, 52349 Düren (DE); Möller, Klaus, Dr., 52249 Eschweiler (DE)
(74) Vertreter: Albrecht, Ralf

(56) Entgegenhaltungen:
- WO-A1-00/09746
- WO-A1-2011/083429
- WO-A2-02/056030
- WO-A2-2007/044427
- US-A- 5 010 183
- "Isolation of Total RNA from Whole Blood and from Cells Isolated from Whole Blood Protocol", , 1. August 2004 (2004-08-01), XP55042655, Gefunden im Internet: URL:http://tools.invitrogen.com/content/sf s/manuals/cms_041270.pdf [gefunden am 2012-10-30]
- "Isolation of Total RNA from Whole Blood, Quick Reference Card, Chemistry on the ABI PRISM 6100 Nucleic Acid PrepStation", , 1. Januar 2002 (2002-01-01), XP55042803, Gefunden im Internet: URL:http://tools.invitrogen.com/content/sf s/manuals/cms_041286.pdf [gefunden am 2012-10-31]
- "SAFETY DATA SHEET, NUCLEIC ACID PURIFICATION LYSIS SOLUTION, PRODUCTNUMBER : 4305895", , 6. September 2009 (2009-09-06), XP55042799, Gefunden im Internet: URL:http://www3.appliedbiosystems.com/cms/ groups/public/documents/msds/cms_065353.pd f [gefunden am 2012-10-31]
- "Total RNA Purification from Whole Blood", APPLICATION NOTE, 1. Februar 2002 (2002-02-01), XP55042806, Gefunden im Internet: URL:http://tools.invitrogen.com/content/sf s/brochures/cms_039875.pdf [gefunden am 2012-10-31]
- "RNA from blood User manual NucleoSpin 8 RNA Blood", , 1. Oktober 2011 (2011-10-01), XP55042645, Gefunden im Internet: URL:http://www.mn-net.com/Portals/8/attach ments/Redakteure_Bio/Protocols/RNA and mRNA/UM_TotalRNABlood_NS8.pdf [gefunden am 2012-10-30]

## Beschreibung

Die vorliegende Erfindung betrifft ein Verfahren zur Isolierung von RNA aus einer Vollblutprobe.

Blut stellt eine zugängliche Quelle für tierische oder humane RNA dar. Blut besteht aus zellulären Bestandteilen und dem Plasma, wobei der zelluläre Anteil je nach Geschlecht und Alter schwankt, im Mittel aber üblicherweise bei 44% liegt. Bedingt durch diesen hohen zellulären Anteil wird Blut häufig auch als "flüssiges Gewebe" bezeichnet.

Trotz dieses hohen zellulären Anteils im Blut ist die Zahl metabolisch aktiver Zellen insgesamt äußerst gering. So machen die DNA- und RNA-tragenden Leukozyten nur etwa 0,1 bis 0,2% der zellulären Fraktion aus, wobei die mit Abstand größte Zellpopulation durch reife Erythrozyten gestellt wird. Diese sind jedoch kernlos, d.h. sie tragen in der Regel keine RNA, dafür aber umso mehr Hämoglobin für den Sauerstofftransport im Blut.

Aufgrund der im Verhältnis zur RNA im Blut stärker vertretenden DNA, ist die Isolierung von genomischer DNA aus Blut verhältnismäßig leicht zu bewerkstelligen. Die hohe Stabilität der DNA ist hierbei ebenfalls von Vorteil. Demgegenüber ist die Isolierung intakter RNA äußerst anspruchsvoll, insbesondere wenn die RNA möglichst vollständig aus der Blutprobe isoliert werden soll. Wie bereits vorangehend angedeutet wurde, ist dies zum einen durch den geringen Anteil RNA-tragender Zellen bedingt.

Einen weiteren Erschwernisfaktor stellt die hohe Konzentration an RNasen im Blut dar, welche sowohl intrazellulär als auch extrazellulär vorliegen. Werden die RNasen nicht möglichst schnell und vollständig inaktiviert, führen diese zu einer Degradierung der RNA und damit einer weiteren Reduzierung der RNA-Konzentration.

Ein weiteres Problem stellt die erforderliche Abtrennung des Hämoglobins dar, da das eisentragende Häm ein äußerst potenter Inhibitor der Polymerasekettenreaktion (polyerase chain reaction, PCR) ist.

Abgesehen von den vorgenannten biochemischen Schwierigkeiten, die sich bei der RNA-Extraktion aus Blutproben ergeben, ist die mitunter sehr hohe Viskosität von Blut problematisch. Vor allem bei hohem zellulären Anteil (Hämatokrit), der beispielsweise krankheitsbedingt auftreten kann, ist Vollblut besonders viskos und daher schwierig zu prozessieren.

Die meisten der heute eingesetzten Verfahren zur Isolierung von RNA aus Vollblut setzen auf die Trennung der RNA-haltigen Leukozyten vom Rest des Blutes mit seinen zellulären und zellfreien Bestandteilen.

Die ursprünglichste Methode, eine reine Leukozytenfraktion zu erhalten, ist die Dichtegradientenzentrifugation (Böyum, 1968; Isolation of mononuclear cells and granulocytes from human blood. Scand. J. Clin. Lab. Invest. 21 (Suppl. 97):77-89). Eine Alternative bieten die s.g. CPT Tubes (Becton, Dickinson and Company, BD Vacutainer CPT Cell Preparation Tube), die ein Polymergel enthalten und nach Blutzugabe und Zentrifugation die Entnahme der Leukozytenfraktion erlauben. Für die RNA-Isolierung und -analyse eignen sich diese aufwändigen Methoden allerdings nicht oder nur sehr eingeschränkt, da das zelluläre RNA-Profil nicht stabilisiert oder "eingefroren" wird. Im Laufe des Prozessierens kommt es unweigerlich zu Veränderungen in der Genexpression, so dass eine Analyse von Expressionsmustern erschwert oder unmöglich gemacht wird. Um die RNA der Proben zu analysieren ist es daher notwendig, diejenigen Faktoren, die die Genexpression beeinflussen, sehr stringent zu kontrollieren. Werden - auch nur für kurze Zeit - unphysiologische Bedingungen geschaffen (z.B. durch Zentrifugation, Sammeln von Zellen, Verdünnen, etc.), so kann eine Beeinträchtigung der Genexpression nicht ausgeschlossen werden.

Eine gängige Methode die Leukozyten anzureichern ist die selektive Erythrozytenlyse (Karavitaki et al., 2005, Molecular staging using qualitative RT-PCR analysis detecting thyreoglubulin mRNA in the periphereal Blood of patients with differentiated thyroid cancer after therapy. Anticancer Research 25: 3135-3142). Diese Methode ist zudem in der EP 0 875 271 und US 5,702,884 beschrieben. Einfach gesagt, werden Erythrozyten durch Zugabe einiger Volumen von Ammoniumchlorid-Lösung oder anderen selektiv wirkenden Formulierungen lysiert, während die Leukozyten unter den gewählten Bedingungen intakt bleiben. Nach Zentrifugation wird das Leukozytenpellet gewaschen, resuspendiert und die RNA aus den Zellen isoliert. Ein großer Nachteil ist die unhandliche Erhöhung des Probenvolumens, einhergehend mit einer weiteren Verdünnung der Probe, da i.d.R. durch Zugabe von mehreren Volumen der Lyselösung hypotonischen Bedingungen eingestellt werden müssen. Auch die wiederholten Zentrifugationsschritte sind zeitaufwändig und nicht zu automatisieren, was ein gravierender Nachteil ist. Durch die unphysiologischen Bedingungen während der Verdünnung kann es zudem zu einer Veränderung der Genexpressionsprofile kommen, die eine spätere Analyse schwierig macht. Der hohe Zeitbedarf dieser Methode fördert diesen Effekt noch weiter. Da während der selektiven Lyse auch die RNasen nicht weiter unterdrückt werden, ist die Qualität und Quantität der isolierten RNA generell niedrig.

Eine andere Möglichkeit, Leukozyten von übrigen Blutbestandteilen zu trennen ist die Isolierung eines s.g. Buffy-Coats. Dabei wird antikoaguliertes Blut bei niedriger Beschleunigung (ca. 2.000 x g) zentrifugiert. Der Buffy-Coat mit überwiegend weißen Blutzellen kann als Schicht zwischen dem Plasma und den gepackten Erythrozyten entnommen werden. Vorteilhaft ist hierbei, dass die Leukozyten unter nahezu physiologischen Bedingungen gesammelt werden, Verdünnungen fehlen hierbei gänzlich. Allerdings ist die Prozessierung zeit- und arbeitsintensiv, bedarf viel praktischer Erfahrung und Geschick und kann Änderungen der Genexpressionsmuster durch Zentrifugation und Prozessierung nicht ausschließen. Hinzu kommt auch das Risiko beim Umgang mit potentiell infektiösem Probenmaterial, ebenso wie die fehlende Möglichkeit, diesen Prozess zu automatisieren.

Eine weitere Möglichkeit, Leukozyten anzureichern beschreibt die US 2005/0208501 A1. Hier wird eine Membran eingesetzt, die selektiv Leukozyten aus Vollblut bindet. Ursprünglich wurden solche Membranen bei Bluttransfusionen eingesetzt um *graft vs. host* Erkrankungen bei den Blutempfängern auszuschließen. Für die Extraktion von RNA aus Vollblut wird dieses über die Membran geleitet (zentrifugiert). Die Leukozyten binden und können anschließend lysiert werden. Im Vergleich zur Dichtegradientenzentrifugation oder der Gewinnung von Buffy-Coat ist diese Methode ungleich einfacher, dennoch kann auch hier nicht eine Veränderung der Genexpressionsprofile ausgeschlossen werden, da die Proben "unphysiologisch" zentrifugiert und Zellen an eine Membran gebunden werden.

Nachteile aller Verfahren, die auf die Trennung von Leukozyten und den restlichen Bestandteilen des Blutes setzen, sind die zeit- und arbeitsintensive Bearbeitung, verbunden mit hohen Kosten. Bei einigen Methoden kommt noch hinzu, dass potentiell gefährliche Erreger im Blut (z.B. Viren) nicht inaktiviert werden und für den Anwender potentiell infektiös bleiben. Ebenso ist die Veränderung der Genexpressionsprofile unerwünscht und kann nicht gänzlich ausgeschlossen werden. Hier wäre eine schnelle, sofortige Lyse hilfreich, die RNasen effizient inaktiviert und damit Veränderung am RNA-Muster ausschließt. Letztlich ist noch die fehlende Möglichkeit der Automation hervorzuheben, da beispielsweise Zentrifugationsschritte nicht, oder nur mit ganz erheblichem Aufwand, auf gängigen Laborautomaten umgesetzt werden können.

Als Alternative zur vorherigen Trennung und Isolierung der Leukozyten kann eine Vollblutprobe auch direkt lysiert werden. Oftmals werden für die Lyse von Vollblut und die Isolierung von RNA Lysepuffer mit Guanidiniumthiocyanat (GuSCN) eingesetzt (Tan and Yiap, "DNA, RNA and Protein Extraction: The Past and The Present", Journal of Biomedicine and Biotechnology, Vol. 2009, Article ID 574398). Das chaotrope Salz führt zur unmittelbaren Lyse der Zellen und gleichzeitig werden RNasen unmittelbar inaktiviert. GuSCN führt auch zur Inaktivierung von Pathogenen, die als potentielle Kontaminationen im Blut vorhanden sind, was die Anwendersicherheit erhöht.

Die EP 0 818 542 A1 beschreibt beispielsweise eine durch pulverförmig eingesetztes GuSCN hervorgerufene Blutlyse. Durch den Kontakt mit Blut wird dieses dann in Lösung gebracht. Durch die zeitlich versetzte Lösung und die anfänglich niedrige Konzentration in der Blutprobe kann nicht ausgeschlossen werden, dass bereits ein Teil der freigesetzten RNA degradiert wird, bevor die effektive Wirkkonzentration des schließlich in Lösung gebrachten GuSCNs ausreichend hoch ist.

In der WO 00/09746 wird ein Gefäß zur Blutentnahme beschrieben, in dem eine wässrige Lösung aus einem Guanidiniumsalz, einer Puffersubstanz, einem Reduktionsmittel und/oder einem Detergenz vorliegt. Wird Blut in dieses Gefäß gefüllt, so werden die Zellen lysiert und die RNA stabilisiert. Nach Lagerung der Blutproben kann die RNA mit gängigen Methoden der Nukleinsäureisolierung gewonnen werden.

Andere Methoden basieren auf der Verwendung von kationischen Detergenzien (Macfarlane, US 5, 010, 183, sowie US 5, 728, 822) oder Lithiumchlorid/Harnstoff.

Kommerzielle Produkte, welche die direkte Lyse zur RNA Isolierung aus Vollblut nutzen, umfassen beispielsweise die PAXgene Tubes von Preanalytix (WO 02/056030). Hier erfolgt die Lyse des Blutes mit den zuvor genannten kationischen Detergenzien. Diese Tubes sind vorrangig für die Stabilisierung von RNA in Blutproben geeignet. So werden die RNAs geschützt, und selbst bei Lagerung für mehrere Tage bei Raumtemperatur (beispielsweise während eines Postversands) bleiben die Expressionsprofile unverändert. Sollen nach Lagerung dann die Nukleinsäuren (RNA) aus den Tubes isoliert werden, so erfolgt zunächst eine Pelletierung durch Zentrifugation. Das Pellet, das die unlöslichen Nukleinsäuren und Proteine enthält, wird dann gewaschen, resuspendiert und anschließend die RNA mit klassischen Methoden (etwa durch Bindung unter Hochsalzbedingungen an Silica-membranen) isoliert.

Andere kommerzielle Tubes zur RNA-Stabilisierung sind die Tempus Blood RNA-Tubes von Applied Biosystems. Hier werden Lösungen enthaltende Guanidiniumhydrochorid (GuHCl), MOPS und NaCl eingesetzt.

Außer zur Stabilisierung der RNA wird die direkte Lyse nur in wenigen Fällen eingesetzt. Im RiboPure Kit (Ambion, LifeTechnologies) wird eine Vollblutprobe mit Hilfe von Phenol/Chloroform lysiert. Bei dieser Methode wird die Probe in Anlehnung an Chomczymski und Sacchi (1987, Single step method of RNA isolation by acid guanidinium thiocyanate-phenol-chloroform extraction. Anal. Biochem, 162(1)) behandelt, und zwar mit einem GuSCN/Phenol/Chloroform Gemisch bei niedrigem pH-Wert. Unter den sauren Bedingungen verbleibt die RNA in der wässrigen oberen Phase, während DNA und denaturierte Proteine in der Interphase bzw. der unteren organischen Phase verbleiben. Üblicherweise wird die RNA dann durch Präzipitation mit Isopropanol entsalzt und gereinigt. Auch wenn einige Nachteile aus dem Stand der Technik mit diesem Ansatz umgangen werden (z.B. sofortige und komplette Lyse) so ist ein ganz wesentlicher Nachteil dieser sehr aufwändigen Methode die Toxizität von Phenol, so dass aus Anwendersicht möglichst auf diese Methoden verzichtet wird. Weiterhin ist die Bearbeitung des 2-Phasensystems praktisch nicht zu automatisieren, so dass der Durchsatz gering bleibt.

Ein weiteres Verfahren liegt dem "Total RNA" Kit (Applied Biosystems) für den ABI Prism 6100 Nucleic Acid Prepstation zugrunde (WO 2005/003346 A1) . Vollblutproben werden in einem ersten Schritt im Verhältnis 1:1 mit PBS (phosphate buffert saline) verdünnt. Diese so vorprozessierten Proben werden dann mit einem GuHCl-haltigen Lysepuffer lysiert und die RNA wird unter Vakuum an eine Membran gebunden. Die Verdünnung mit PBS führt zum einen zu einer unhandlichen Vergrößerung des Volumens und bietet wie alle Methoden, bei denen die Blutprobe vor der Lyse konditioniert und verändert wird, die Gefahr, dass sich die Genexpression aufgrund der unphysiologischen Bedingungen ändert. Die Verdünnung resultiert vermutlich aus der Notwendigkeit heraus, die Probe auch in Ihrer Viskosität zu kontrollieren. Bedingt durch das Prozessieren unter Vakuum bestünde ansonsten die Gefahr, dass die Membranen verstopfen.

Die direkte Lyse von Blut wird auch in der US 7,927,798 beschrieben. Hier muss die Lyse so durchgeführt werden, dass eine Inhibition der RNA-Amplifikation verhindert wird. Anders als bei herkömmlichen PCR-basierten Verfahren, wird die RNA mittels der s.g. bDNA (branched DNA)-Technologie amplifiziert. Dabei wird die RNA mittels Sonden an einer festen Phase immobilisiert und durch repetitive, sequentielle Hydridisierung mit weiteren Sonden amplifiziert. Die Detektion erfolgt schließlich über Chemilumineszenz. Bedingt durch den direkten Nachweis ohne Aufreinigung ist die eingesetzte Blutmenge limitiert. Diese schwankt zwischen 1 und 30 µL bei einem finalen Volumen von 150 µL. Die Probe wird also um einen Faktor von 5-150 verdünnt.

Bedingt durch die folgende Hybridisierung der RNA verbieten sich hochmolare Lyselösungen oder andere Formulierungen, die die Bindung und Hybridisierung stören. Nachteilig ist dabei zudem die Limitierung des Probenmaterials, da nur kleinste Probenmengen eingesetzt werden und die damit verbundene geringe Sensitivität und die Gefahr der Inhibition der Folgeanalytik, da ohne die übliche Reinigung der Nucleinsäuren nicht sichergestellt werden kann, dass jedwede Inhibitoren entfernt worden sind.

In der WO 2011/083429 A1 ist ein Verfahren zur Isolierung von RNA aus einer Vollblutprobe offenbart, bei der ein Lysepuffer verwendet wird, der eine Lysesubstanz, Proteinase K und ein Detergenz enthält. Dabei wird auch Magnesiumchlorid zur Deaktivierung von RNasen verwendet. Auch hier ist das Verhältnis zwischen Gesamtvolumen und Probe sehr hoch, verbunden mit den oben beschriebenen Nachteilen einer solch großen Verdünnung.

Der vorliegenden Erfindung liegt die Aufgabe zugrunde, ein Verfahren zur Isolierung von RNA aus Vollblutproben zur Verfügung zu stellen, das die unmittelbare Prozessierung der Proben erlaubt und insbesondere ohne vorherige Stabilisierung der Nucleinsäuren durchgeführt werden kann und dabei eine möglichst vollständige Isolierung der im Probenmaterial enthaltenen RNA ermöglicht. Das Verfahren soll zudem die Möglichkeit zur automatisierten Probenverarbeitung bieten.

Gelöst wird diese Aufgabe durch ein Verfahren zur Isolierung von RNA aus einer Vollblutprobe, umfassend die Schritte
a) Inkontaktbringen der Probe mit einer wässrigen Lyse-Lösung enthaltend wenigstens eine Lysesubstanz in einer Konzentration von 1,5 Mol/l bis 7 Mol/l, wenigstens ein Detergenz und Magnesiumionen,
b) zeitgleiche oder anschließende Zugabe einer Proteinase, insbesondere Proteinase K,
c) danach Inkubieren der nach Schritt b) erhaltenen Lösung zum wenigstens teilweisen enzymatischen Verdau und Lyse der Probe wobei ein Lysat gewonnen wird und
d) Isolierung der RNA aus dem Lysat,
wobei die Probe im Schritt a) unmittelbar mit der Lyse-Lösung in Kontakt gebracht wird und bis zum Abschluss des Schritts c) nicht durch Zugabe eines weiteren Lösungsmittels verdünnt, kein Stabilisator zugegeben und/oder nicht zentrifugiert wird, wobei das Gesamtvolumen aus Probe, wässriger Lyse-Lösung und Proteinase bis zum Abschluss des Schritts c) das Ausgangsvolumen der Probe um nicht mehr als den Faktor 2,5 übersteigt, vorzugsweise um nicht mehr als den Faktor 1,5.

Es hat sich überraschenderweise gezeigt, dass durch kombinierten Einsatz einer Lyse-Substanz, die wenigstens ein Detergenz und Magnesiumionen enthält, in den genannten Konzentrationsbereichen sowie die gleichzeitige oder nachfolgende Zugabe einer Proteinase eine vollständige Lyse von Vollblutproben erzielbar ist, wobei die erfindungsgemäßen Konzentrationen an Lysesubstanz die Aktivität der Proteinase nicht wesentlich beeinflussen, so dass auch hochviskose Vollblutproben automatisiert prozessiert werden können. Mit Hilfe der letztgenannten Maßnahmen kann die Komplexität des erfindungsgemäßen Verfahrens begrenzt werden, was dessen Fehleranfälligkeit reduziert und die Robustheit der Methode und die Möglichkeit zur Automatisierung weiter verbessert.

Gleichzeitig ist die Lyse-Substanz-Konzentration ausreichend hoch, um die in der Probe vorhandenen RNasen in kürzester Zeit zu inaktivieren. Weiterhin ermöglicht das mittels des erfindungsgemäßen Verfahrens erhaltene Lysat eine Bindung der RNA an eine feste Phase unter üblichen Bedingungen, wie beispielsweise durch Zugabe von Ethanol.

Durch den erfindungsgemäß limitierten Einsatz flüssiger Chemikalien wird zudem bei der Probenverarbeitung das Gesamtvolumen der Probe nicht in dem Maße vergrößert, wie dies teilweise bei den aus dem Stand der Technik bekannten Verfahren der Fall ist, die häufig sogar eine Verdünnung der Probe vor deren eigentlicher Prozessierung vorsehen. Auf diese Weise kann das erfindungsgemäße Verfahren in verhältnismäßig kleinen Reaktionsgefäßen durchgeführt werden, was die automatisierte Probenverarbeitung erheblich vereinfacht.

Unter Isolierung der RNA wird vorliegend verstanden, dass die RNA von den übrigen Probenbestandteilen, insbesondere von Proteinen, Zellbruchstücken und dergleichen, weitestgehend befreit wird. Die Entfernung weiterer Nukleinsäuren wie DNA ist hingegen optional und richtet sich danach, ob diese bei der weiteren Verarbeitung oder Analyse der RNA stören. Allgemein ist das erfindungsgemäße Verfahren einfach durchzuführen und, wie bereits voranstehend erörtert, ohne weiteres automatisierbar. So ermöglicht das erfindungsgemäße Verfahren die Vollblutlyse unter Wegfall sämtlicher Zentrifugationsschritte, wie sie teilweise bei bislang bekannten Verfahren vorgesehen sind. Weiterhin benötigt das erfindungsgemäße Verfahren keine vorherige Vorbehandlung oder Konditionierung der Proben, was nicht nur den Verarbeitungsaufwand reduziert, sondern auch die Gefahr möglicher Verunreinigung mindert. In bevorzugter Weise wird also die Probe vor dem Inkontaktbringen mit der wässrigen Lyse-Lösung keinem vorangehenden Prozessschritt unterworfen.

Ein weiterer Vorteil der vorliegenden Erfindung besteht darin, dass das Verfahren ohne den Einsatz toxischer Formulierungen wie Phenol/Chloroform durchgeführt werden kann. Das erfindungsgemäße Verfahren ist zudem vergleichsweise unempfindlich gegenüber Schwankungen im Hämatokrit, so dass die RNA-Extraktion ohne die Gefahr von Verstopfungen mit üblichen Methoden unter Verwendung zur RNA-Isolierung geeigneter Membranen prozessiert werden kann. Das erfindungsgemäße Verfahren ermöglicht zudem die Extraktion von RNA aus Vollblut in hoher Qualität und Ausbeute.

Die mit Hilfe des erfindungsgemäßen Verfahrens isolierbare RNA umfasst im Prinzip sämtliche im Vollblut vorkommende RNA, wie beispielsweise mRNA, rRNA sowie miRNA, um nur einige zu nennen.

Als erfindungsgemäß eingesetzte Proteinasen kommen beispielsweise Proteinase K, oder andere Proteinase mit breitem Substratspektrum (bevorzugt mit Endo- und/oder Exopeptidaseaktivität, sowie deren Mischungen in Frage, wobei Proteinase K besonders bevorzugt ist, da hierdurch die Viskosität der Vollblutprobe besonders effizient reduziert werden kann. Die Mengen an zugesetzter Proteinase können beispielsweise bei 0,1 - 5 mg/ ml Vollblut liegen, vorzugsweise bei 0,5 bis 1 mg/ ml.

Gemäß einer bevorzugten Ausführungsform des erfindungsgemäßen Verfahrens werden im Schnitt c) in der Lösung enthaltene RNasen weitestgehend vollständig inaktiviert. Dies wird mit Hilfe der erfindungsgemäß vorgesehenen Konzentrationen der Lysesubstanz bewerkstelligt.

Als Maß für die Inaktivierung von RNasen kann die Integrität und Stabilität der Isolierten RNA herangezogen werden. Nur wenn RNasen während der Lyse und dem Probenaufschluss effizient und weitestgehend inaktiviert werden, zeigt die isolierte RNA bei Analyse mittels denaturierender Gelelektrophorese oder mittels RNA-Assay auf dem Agilent Bioanalyzer distikte rRNA-Banden bekannter Größe. Bei humanen Blutproben erscheint die RNA (18 und 28S rRNA) als zwei distinkte Banden, von denen die 28S rRNA deutlich stärker ausgeprägt ist. Da RNA gegenüber den ubiquitären RNasen sehr empfindlich ist, wird empfohlen, isolierte RNA einzufrieren oder zumindest bei 4°C zu lagern. Wird isolierte RNA (zu erkennen an klaren 18 und 28S rRNA Banden) z.B. für einen Tag bei Raumtemperatur gelagert, erneut mittels RNA-Gel oder Bioanalyzer analysiert, so kann bei Vorliegen der beiden distinkten Banden auf die weitestgehend vollständige Abwesenheit von RNasen geschlossen werden. Wäre die isolierte RNA mit RNasen kontaminiert, so würde die RNA durch die Inkubation bei Raumtemperatur abgebaut; die Unterschiede auf dem Gel wären augenfällig.

In weiter bevorzugter Weise enthält die wässrige Lyse-Lösung die Lysesubstanz in einer Konzentration von 2 Mol/l bis 6 Mol/l, insbesondere von 2, 5 Mol/l bis 5 Mol/l, bevorzugt von 3 Mol/l bis 4 Mol/l. Diese Konzentrationen sind besonders vorteilhaft, weil diese einerseits ausreichend hoch sind, die vorhandenen RNasen zügig und vollständig zu inaktivieren, bevor diese die vorhandene RNA abbauen. Andererseits sind diese Konzentrationen so gewählt, dass es nicht zu einer stärkeren Inhibition der Proteinase kommt, so dass die Probenviskosität während der Inkubation in ausreichendem Maße reduziert wird, sodass die Möglichkeit zur automatisierten Probenverarbeitung bestehen bleibt.

Das dem erfindungsgemäßen Verfahren zugeführte Vollblut kann humanen Ursprungs sein oder von anderen Spezies stammen, wie etwa von Tieren. Das Blut kann zudem frisch entnommen oder in gefrorener Form vorliegen und aus handelsüblichen Entnahmesystemen mit üblichen Antikoagulantien (EDTA, Zitrat, Heparin) stammen.

In weiter bevorzugter Weise wird die wässrige Lyse-Lösung mit der Probe in einem Volumenverhältnis von 1,8 : 1 bis 1 : 1,8 vermischt wird, insbesondere von 1,5 : 1 bis 1 : 1,5, vorzugsweise von 1,2 : 1 bis 1 : 1,2. Besonders bevorzugt liegt das vorgenannte Mischungsverhältnis bei etwa 1:1. Die vorgenannten Mischungsverhältnisse sind besonders vorteilhaft, da hierdurch eine unnötige Erhöhung des Probenvolumens durch die eingesetzten Lösungen vermieden werden kann.

Nach einer weiteren vorteilhaften Ausgestaltung des erfindungsgemäßen Verfahrens wird im Schritt b) die Proteinase als Feststoff zusammen mit einem Lösungsmittel oder als Proteinase-Lösung zugesetzt, wobei die jeweilige Flüssigkeitsmenge so eingestellt wird, dass die Flüssigkeitsmenge höchstens 20 % des Volumens aus Probe und wässriger Lyse-Lösung beträgt, insbesondere höchstens 10 %. Durch die vorgenannte Maßnahme wird ebenfalls eine unnötige Verdünnung der Probe und Vergrößerung des Probenvolumens vermieden, was sich vorteilhaft auf die Automatisierungsmöglichkeit des erfindungsgemäßen Verfahrens auswirkt.

Für die im Rahmen des erfindungsgemäßen Verfahrens einsetzbaren Lyse-Substanzen kommt eine Reihe von Verbindungen in Betracht. Dabei wird unter einer Lysesubstanz im Sinn der vorliegenden Erfindung eine Verbindung verstanden, die in der Lage ist, Biomoleküle aus der Vollblutprobe freizusetzen und zwar zumindest die RNA. So kann die Lysesubstanz ein Chaotropsalzoder Mischungen verschiedener Chaotropsalze enthalten. Dabei sind die Chaotropsalze insbesondere ausgewählt aus Thiocyanaten, wie Natriumthiocyanat, Kaliumthiocyanat und Guadiniumthiocyanat, Harnstoff, Perchloratsalzen wie Natriumperchlorat und/oder Kaliumiodid oder Mischungen hiervon. Von den vorgenannten Verbindungen sind Thiocyanate bevorzugt, weil diese Verbindung in besonderer Weise in der Lage ist, RNasen zu deaktivieren, wobei Guadiniumtiocyanat besonders bevorzugt ist.

Gemäß einer besonders bevorzugten Ausführungsform des erfindungsgemäßen Verfahrens enthält die Lyse-Lösung Magnesiumionen in einer Konzentration von 10 bis 1000 mMol/l, bevorzugt von 100 bis 600 mMol/l, weiter bevorzugt 150 bis 400 mMol/l. Der Zusatz von Magnesiumionen ist besonders vorteilhaft, weil auf diese Weise die Ausbeuten an RNA gesteigert werden können.

In ebenfalls bevorzugter Weise kann vorgesehen sein, dass die Lyse-Substanz Kalium- und/ oder Natriumthiocynat umfasst und die Lyse-Lösung Calciumionen enthält, wobei die Konzentration an Calciumionen insbesondere 10 bis 150 mMol/l beträgt, bevorzugt von 50 bis 100 mMol/l. Auch durch diese Kombination kann die RNA-Ausbeute des erfindungsgemäßen Verfahrens gesteigert werden, wobei auch der gleichzeitige Einsatz mit Magnesiumionen wie oben beschrieben möglich ist.

Als Quelle für Magnesiumionen und Calciumionen kommen im Prinzip sämtliche wasserlöslichen Salze der vorgenannten Metalle in Frage, sofern die Kationen dieser Salze unter den Verfahrensbedingungen chemisch und biologisch inert sind. Dies gilt beispielsweise für die Chloride von Magnesium und Calcium.

In weiter bevorzugter Weise ist die Lyse-Lösung frei von weiteren zwei- oder dreiwertigen Metallionen, d.h. abgesehen von Magnesium- bzw. Calciumionen. Dies ist besonders vorteilhaft, weil sich herausgestellt hat, dass zwar Magnesium- bzw. Calciumionen einen vorteilhaften Einfluss auf die RNA-Ausbeute haben, jedoch andere mehrwertige Metallionen in der Regel zu einer Ausfällung von Probenbestandteilen führen. Dies hat eine erhebliche Verringerung der RNA-Ausbeute zur Folge und erschwert zudem die automatisierte Probenaufbereitung, da beispielsweise für die Isolation verwendete Membranen durch die ausgefällten Bestandteile verstopft werden.

Erfindungsgemäß ist vorgesehen, dass die Lyse-Lösung ein Detergenz enthält. Hierbei können im Prinzip sämtliche für biochemische Extraktionsprozesse von Zellkernbestandteilen verwendbare Detergenzien zur Anwendung kommen, wobei nicht-ionische Detergenzien, sowie Mischungen von diesen besonders bevorzugt sind. Das Detergenz unterstützt die Lyse, löst Lipide der lysierten Blutzellen und verhindert auf diese Weise ein Verstopfen von zur Abtrennung verwendeten Membranen. Besonders vorteilhaft auf die Qualität der extrahierten RNA und deren Ausbeute wirkt sich der Einsatz von Polyoxyethylen-20-Cetylether (Brij 58®), N-Lauroyl-Sarkosin sowie deren Mischungen aus. Auch andere Detergenzien, wie beispielsweise Tween20 können alleine oder in Mischungen mit den vorgenannten Detergenzien verwendet werden.

Die Mengen an eingesetzten Detergenzien können über weite Bereiche variiert werden. So kann die Lyse-Lösung beispielsweise einen Gesamtgehalt an Detergenzien von 10 bis 200 g/l enthalten, vorzugsweise von 30 bis 100 g/l.

Die erfindungsgemäß eingesetzte Lyse-Lösung kann neben den vorgenannten Inhaltsstoffen noch weitere Bestandteile beinhalten, die insbesondere ausgewählt sind aus Puffersubstanzen, Enzymen, Reduktionsmittel und/oder Alkohole.

Die Isolation der RNA aus dem Lysat in Schritt d) kann prinzipiell auf jede an sich bekannte Weise vorgenommen werden. In vorteilhafter Weise wird die RNA zur Isolierung aus dem Lysat an eine feste Phase angelagert, gewünschtenfalls gefolgt von einem oder mehreren Waschschritten.

Unter festen Phasen werden wasserunlösliche Materialien verstanden, an welche Nucleinsäuren in wässriger Phase in Gegenwart hoher Ionenstärke binden. Dies sind beispielsweise poröse oder nicht-poröse Mineralpartikel wie Silica, Glas, Quarz, Zeolithe oder Mischungen hiervon. Die vorgenannten Partikel können zudem als magnetische Partikel ausgebildet sein. Bei der Verwendung der vorgenannten magnetisch modifizierten Partikel, die auch als Magnetic-Beads bekannt sind, kann auf eine Magnetseparation zurückgegriffen werden. Derartige Verfahrensweisen sind dem Fachmann prinzipiell bekannt. Im Übrigen wird bei der Verwendung nicht-magnetischer Partikel eine Separation durch Sedimentation oder Zentrifugation vorgenommen.

Die feste Phase kann in Form eines sedimentierenden Pulvers oder einer Suspension vorliegen oder aber in Form einer Membran, einer Filterschicht, einer Fritte, eines Monolithen oder eines anderweitigen festen Körpers ausgebildet sein. Von den vorgenannten möglichen Ausführungsformen sind für das erfindungsgemäße Verfahren Silicamembranen oder Glasfaservliese besonders bevorzugt. Der Transport der vorbehandelten Lösung durch diese Materialien kann alleine durch Gravitation oder durch Zentrifugation sowie auch durch Anlegen eines Unterdrucks erfolgen.

Bei besonders bevorzugten Vertretern wird die vorgenannte feste Phase in Form einer Membran oder eines Vlieses in einem Hohlkörper mit einer Ein- und Auslassöffnung ein- oder mehrschichtig fixiert. Solche Hohlkörper sind dem Fachmann beispielsweise als Minispin-Zentrifugenkörper bekannt. So können bei den Minispin-Zentrifugenkörpern bzw. Minispin-Säulen die Binde-, Wasch-, Separations- und Elutionsschritte durch Zentrifugalkraft oder Vakuum vermittelt werden.

In weiterer bevorzugter Weise kann die Anlagerung der RNA an die feste Phase dadurch unterstützt werden, dass dem Lysat ein Bindungsmittel zugegeben wird. Hierfür kommen beispielsweise ein Alkohol, vorzugsweise Ethanol und/oder Isopropanol, sowie eine entsprechende Alkohol/ Wasser Mischung, wie eine Ethanol/Wassermischung in Frage. Der Ethanolgehalt kann insbesondere zwischen 50 und 95 Vol.-% liegen, beispielsweise bei 70 Vol.-% Ethanol.

Im Rahmen des erfindungsgemäßen Verfahrens kann zudem vorgesehen sein, dass die DNA zumindest teilweise, bevorzugt nahezu vollständig entfernt wird, insbesondere durch einen DNase-Verdau. Ob die DNA entfernt wird oder nicht, richtet sich danach, ob diese störend bei der Folgeanalytik ist. Die DNA-Entfernung kann zu einem beliebigen Zeitpunkt erfolgen, nachdem die Lyse der Probe durchgeführt wurde. Zweckmäßig ist es beispielsweise, die DNA nach der Anlagerung der RNA an einen festen Träger enzymatisch abzubauen, also einem DNase-Verdau zu unterziehen. Denn typischerweise bindet die DNA unter den Bindungsbedingungen der RNA ebenfalls, zumindest teilweise, an dem festen Träger, auf dessen Oberfläche die DNA anschließend selektiv abgebaut werden kann.

Der DNase Verdau kann während des Reinigungsverfahrens, z.B. nach Bindung der Nucleinsäuren an eine feste Phase erfolgen (s.g. "on-column" Verdau), oder aber nach Isolierung der RNA in Lösung. Der Nachweis der DNA erfolgt üblicherweise mittels PCR. Eine vollständige Entfernung der DNA, so dass auch mittels sehr kurzer Fragmente von multi-copy Targets keinerlei PCR Amplifikation erfolgt, ist technisch nahezu unmöglich, allein bedingt durch die Tatsache, dass es bestimmte DNA Sequenzen und -spezies gibt, die mittels DNase Verdau nicht degradiert werden können. Dennoch ist die DNA-Entfernung mittels DNase Verdau signifikant: So führt der DNase Verdau während der RNA Isolierung aus Vollblutproben üblicherweise zu einer Verschiebung der cp-Werte in der real-time PCR um 8 - 10 Zyklen. Bedingt durch den halblogarithmischen Zusammenhang zwischen cp-Wert und DNA-Konzentration bedeutet dies etwa eine Abreicherung um den Faktor 250 - 1000.

Zur Gewinnung der isolierten RNA können an sich bekannte Vorgehensweisen zur Anwendung kommen. So kann die isolierte RNA beispielsweise von der festen Phase eluiert bzw. desorbiert werden. Hierzu kann eine Elutionslösung verwendet werden, beispielsweise RNase-freies Wasser oder nur leicht gepufferte wässrige Lösungen, wie etwa 5 m Tris/HCl, pH 8, 5.

Ein weiterer Gegenstand der vorliegenden Erfindung betrifft die Verwendung eines Kits zur Isolierung von RNA aus einer Vollblutprobe, enthaltend zumindest die Bestandteile:
a) eine wässrige Lyse-Lösung enthaltend wenigstens eine Lysesubstanz in einer Konzentration von 1 Mol/l bis 5 Mol/l,
b) wenigstens ein Detergenz,
c) Magnesiumionen,
d) eine Proteinase, insbesondere Proteinase K,

Die vorliegende Erfindung betrifft zudem die Verwendung des erfindungsgemäßen Kits zur Isolierung von RNA aus einer Vollblutprobe.

Die vorliegende Erfindung wird im Folgenden anhand mehrerer Ausführungsbeispiele näher erläutert.

### Beispiel 1:

### Lyse von Vollblutproben und Isolierung der RNA im Minispin Format mit Silica-Membranen

Zur Lyse der Blutproben werden 200 µL Vollblut mit 200 µL Lysepuffer versetzt und kräftig gemischt. Eine erfindungsgemäße Zusammensetzung des RNA Blood Lysepuffers sieht wie folgt aus:
3 M GuSCN,
5% Brij 58 (w/v),
0,015% N-Lauroyl-Sarkosin,
100 mM MgCl₂.

Anschließend wird Proteinase K (20 mg/mL Stammlösung) hinzugegeben, es wird für 15 min bei RT inkubiert. Diese erfindungsgemäße Vorgehensweise wird im Folgenden als direkte Lyse bezeichnet.

### Isolierung der freigesetzten RNA

Zur Isolierung der freigesetzten RNA werden durch Zugabe von 200 µL 70% Ethanol (siehe Schritt 9) die Bedingungen so eingestellt, dass die RNA an eine Silicamatrix binden kann. Die weitere Prozessierung (ab Schritt 12) inkl. eines DNA-Verdaus erfolgt mit dem kommerziell verfügbaren RNA Reinigungskit der Fa. MACHEREY-NAGEL, NucleoSpin RNA II, REF 740955. Dieses Kit enthält die folgenden Bestandteile:
Lysis Buffer RA1 (hier nicht notwendig)
Wash Buffer RA2
Wash Buffer RA3 (concentrate)
Membrane Desalting Buffer MDB
Reaction Buffer for rDNase
rDNase, lyophilized
RNase-free water
NucleoSpin Filter Columns (hier nicht notwendig)
NucleoSpin RNA II Columns
Collection Tubes
User Manual
Wash Buffer RA2 enthält GuSCN sowie Alkohol, Wash Buffer RA3 enthält 80% Ethanol, Rest Wasser und Puffersubstanzen.

Alternativ zu 200 µL Probe können auch abweichende Mengen prozessiert werden. Die Verhältnisse zwischen der Blutprobe, dem RNA Blood Lysis Puffer und dem Ethanol betragen 1:1:1. Bei einem doppelt so hohen Blutvolumen wird auch die Menge an Lysepuffer und Ethanol verdoppelt.

Ein schematischer Ablauf des Verfahrens ist in der folgenden Tabelle dargestellt:

| **Schritt** | |
|---|---|
| 1 | 200 µL Vollblut in 1,5 mL Tube |
| 2 | 200 µL RNA Blood Lyse Puffer |
| 3 | Mix durch 3x invertieren |
| 4 | 20 µL Proteinase K (ca. 20 mg/mL) |
| 5 | mix (vortex) |
| 6 | 15 min bei Raumtemperatur inkubieren auf dem Schüttler: Eppendorf Thermoshake, 1400 rpm |
| 7 | Kurze Zentrifugation (1 s, 2000xg) |
| 8 | Zugabe von 200 µL 70% Ethanol. |
| 9 | Stark schütteln |
| 10 | Kurze Zentrifugation (1 s, 2000xg) |
| 11 | Aufarbeitung mit dem NucleoSpin RNA II Kit. 700 µL des Lysates in eine NucleoSpin RNA II Säule im Collection Tube pipettieren |
| 12 | 30 s bei 11000xg zentrifugieren |
| 13 | Durchfluss und Collection Tube verwerfen |
| 14 | Die Säule in neues Collection Tubes einstecken |
| 15 | Zugabe von 350 µL MDB (Membrane Desalting Buffer) |
| 16 | 1 min bei 11000xg zentrifugieren |
| 17 | Zugabe von 95 µL rDNase |
| 18 | Bei RT für 15 min inkubieren |
| 19 | Zugabe von 200 µL RA2 |
| 20 | 30 s bei 11000xg zentrifugieren |
| 21 | Durchfluss und Collection Tube verwerfen |
| 22 | Die Säule in ein neues Collection Tube einstecken |
| 23 | Zugabe von 600 µL RA3 |
| 24 | 30 s bei 11000xg zentrifugieren |
| 25 | Durchfluss verwerfen und Säule erneut in das Collection Tube stecken |
| 26 | Zugabe von 250 µL RA3 |
| 27 | 2 min bei 11000xg zentrifugieren |
| 28 | Einstecken der Säule in ein Nuclease-freies Collection Tube (1.5 mL). |
| 29 | Zugabe von 60 µL RNase-freiem Wasser |
| 30 | 1 min bei 11000xg zentrifugieren |

Das Ablaufschema zeigt exemplarisch die Bearbeitung von 200 µL Vollblutproben, kann aber auf beliebige Volumina hochskaliert werden. Dazu werden die Volumina von Lysepuffer, Proteinase und Ethanol um den gleichen Faktor erhöht, so dass vergleichbare Lyse- und Bindebedingungen erhalten werden.

### Analyse der isolierten RNA

Für die Beurteilung der Menge und Qualität von RNA können eine ganze Reihe von Untersuchungsmethoden herangezogen werden. Die RNA-Quantifizierung erfolgt über Absorptionsmessungen bei 260 nm, die Qualität wird anhand des Quotienten A260/280 und A260/228 bestimmt. Diese Methode ist in Short protocols in molecular biology. Ed. F. M. Ausubel. 1999 Wiley beschrieben.

Entscheidendes Merkmal für die Qualität einer isolierten RNA ist auch deren Integrität. Diese wird anhand von Bioanalyzer Messungen (Agilent 2100 Bioanalyzer) bestimmt, die vom System generierte RNA-Integrity-Number als Maß der RNA Qualität herangezogen. Die RNA-Integrity-Number, RIN, ist ein Maß für die Qualität der RNA und reicht von idealen 10.0 bis 0.

Weiterhin wird die Eignung der isolierten RNA für die Folgeanalytik anhand von RT-PCR Experimenten überprüft. Hierzu wird eine 73 nt RNA-Sequenz revers traskribiert und mittels PCR amplifiziert. Die Analyse erfolgt im Roche LightCycler mittels real-time PCR. Werden Blutbestandteile, wie etwa das Häm, verschleppt, so kommt es zu einer PCR-Inhibition.

### Ergebnisse:

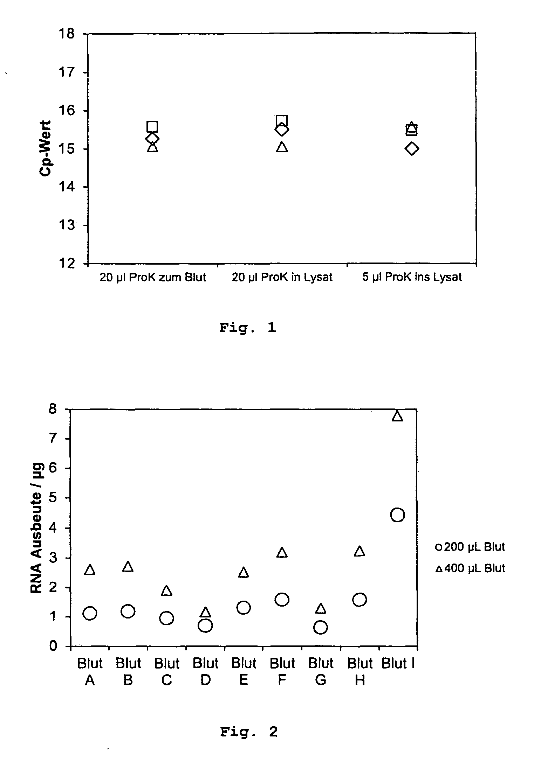

Ob bei dem oben beschriebenen Ablauf die Proteinase K vor der Zugabe des Lysepuffers zur Blutprobe gegeben wird, oder ob diese dem Blut/Lysepuffergemisch zugegeben wird, hat keinen Einfluss auf das Ergebnis. In Fig. 1 ist ein Vergleich zwischen beiden Verfahren gezeigt:
Ansatz 1 (links): Proteinase K wurde direkt zur Blutprobe gegeben.
Ansatz 2 (mitte) und 3 (rechts): Die Blutprobe wurde mit Lysepuffer gemischt, anschließend wurde die Proteinase zugegeben (Ansatz 2 mit 20 µL Proteinase K (20 mg/mL). Ansatz 3: mit 5 µL Proteinase K (20 mg/mL)).

Gezeigt sind jeweils die Einzelwerte von 3 Blutproben (Dreieck, Raute, Quadrat). Die RNA-Menge ist hier als Cp-Wert nach real-time RT-PCR dargestellt. Die Ergebnisse zeigen zudem, dass die Menge der eingesetzten Proteinase nur eine untergeordnete Bedeutung hat. Selbst eine Variation um den Faktor 4 liefert vergleichbare Cp-Werte.

Dass die RNA-Ausbeute mit steigender Blutmenge steigt, ist in der Fig. 2 gezeigt. Die Darstellung illustriert die Ergebnisse der Isolierung von RNA aus Vollblutproben durch direkte Lyse unter Verwendung von mit Minispin-Säulen. Dargestellt ist ein Vergleich der RNA Ausbeute bei 200 und 400 µL Vollblut. Die Bestimmung der RNA-Menge erfolgte über Spektrophotometrische Bestimmung bei 260 nm. Blut A-I: Frischblut, Einzelspender A-I, EDTA stabilisiert.

Sichtbar sind zu erwartende Schwankungen zwischen individuellen Proben, in allen Fällen ist die Ausbeute bei Verwendung von 400 µL Probe größer als bei 200 µL Probe. Auch kann man erkennen, dass sich ebenso wie im Probenvolumen auch in der RNA Ausbeute der Faktor 2 erkennen lässt. Bei der Vielzahl der lysierten Proben kam es in keinem Fall zu einer Verstopfung der Säulen, selbst nicht in Fällen mit sehr hohem Hämatokrit und entsprechend hohen RNA Ausbeuten (vergl. Blut I).

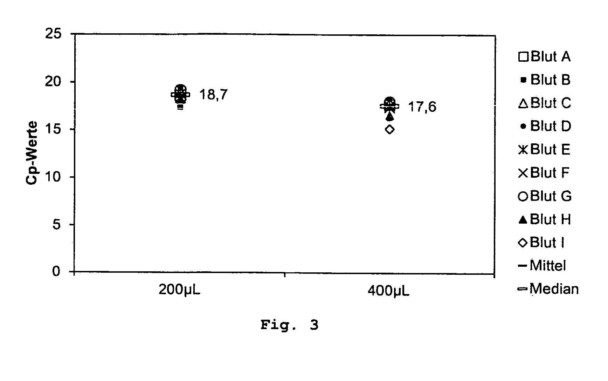

Der Unterschied in der Ausbeute bei Verwendung von 400 statt 200 µL Vollblut zeigt sich auch in der Analyse mit real-time RT-PCR. Wie die Fig. 3 zeigt, führt der Einsatz von 400 µL Blut zu einer Cp-Wert-Reduktion um ca. 1 Zyklus. Die Proben Blut A-I sind Frischblut von Einzelspendern A-I, EDTA stabilisiert. Darstellung der Einzelwerte, sowie Mittel und Median.

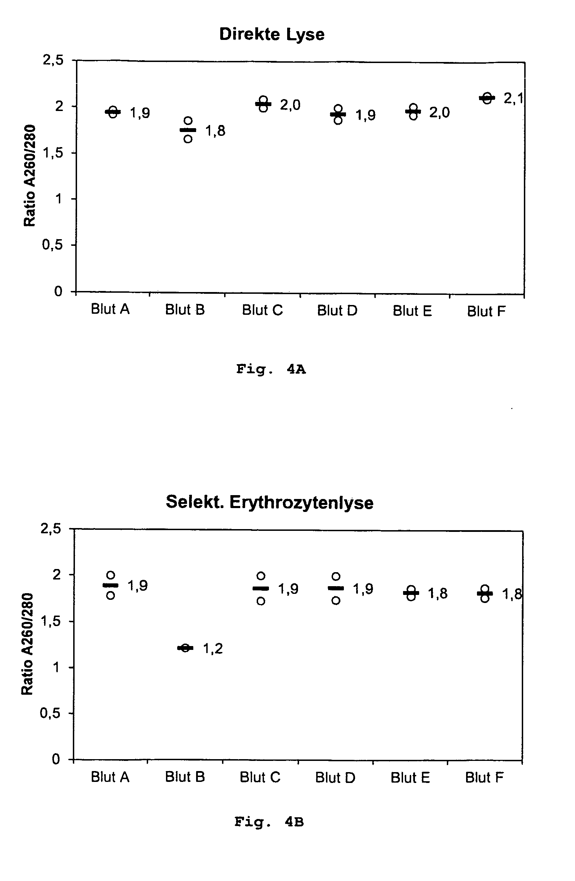

Durch den halblogarithmischen Zusammenhang zwischen Cp-Wert und RNA-Konzentration entspricht die Abnahme um einen Zyklus einem Konzentrationsanstieg um den Faktor 2. Dies ist genau der bei einer Verdopplung der Blutmenge zu erwartende Faktor. Die Qualität der isolierten RNA, gemessen am Ratio A260/280, ist in den Fign. 4A (Isolierung von RNA aus Vollblutproben durch direkte Lyse) und 4B (selektive Erythrozytenlyse) dargestellt. Den Ergebnissen liegt die Prozessierung von jeweils 400 µL Vollblut unter direkter Lyse gemäß Ausführungsbeispiel 1 zugrunde.

Die selektive Erythrozytenlyse wurde mit dem Qiagen-Kit, QIAamp RNA Blood Mini Kit, gemäß beiliegendem Handbuch durchgeführt. Bei den Proben Blut A-F handelt es sich wiederum um Frischblut von Einzelspendern A-F, EDTA stabilisiert. Darstellung der Einzel- (n=2) und Mittelwerte. Idealerweise sollte RNA Ratios A260/280 um 2.0-2.1 aufweisen. Generell ist die Qualität aus Blut isolierter RNA jedoch niedriger als aus anderen Geweben oder Zellen. Die mit dem erfindungsgemäßen Verfahren bestimmten Ratios liegen nahezu ideal zwischen 1.8 und 2.1.

Zum Vergleich sind in der Fig. 4B Ratios gezeigt, die mit einem kommerziellen Kit basierend auf selektiver Erythrozytenlyse gewonnen wurden (Qiagen QIAamp RNA Blood Mini Kit). Auch hier ist die Qualität gut, Ratios über 1.9 werden allerdings nicht erreicht. Auffällig auch die Probe 2, die mit direkter Lyse ein Ratio von 1, 8 lieferte, mit selektiver Erythrozytenlyse jedoch nur von 1,2. Bedingt durch das aufwändige und komplexe Handling bei der selektiven Lyse können Verunreinigungen der RNA nicht ausgeschlossen werden.

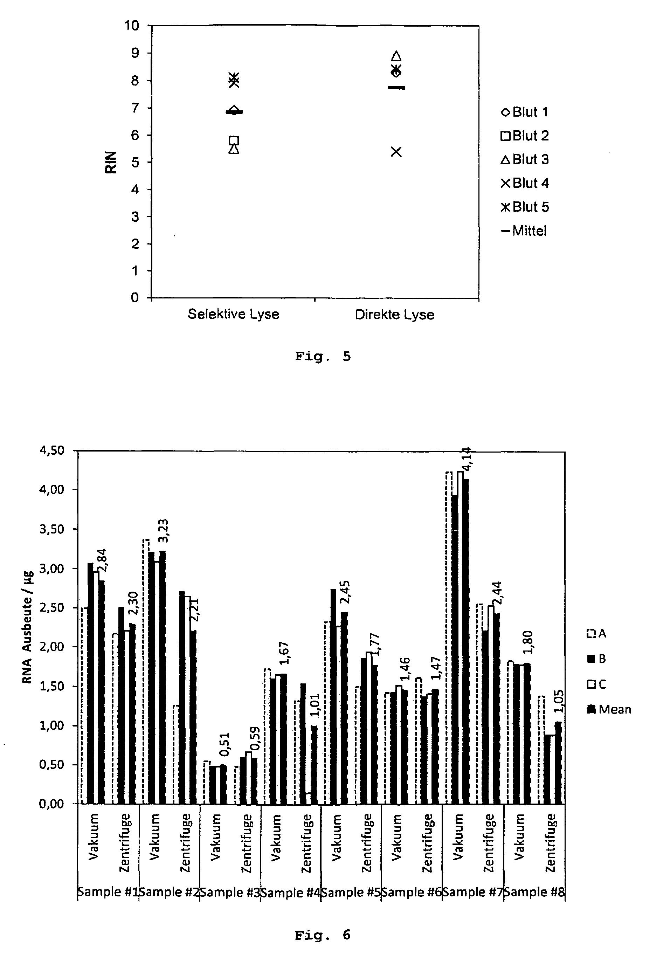

Die mit dem erfindungsgemäßen Verfahren erzielbare RNA-Integrität ist vergleichsweise hoch und zumindest mit Standardverfahren, die auf der selektiven Lyse von Erythrozyten basieren, vergleichbar. In der Fig. 5 sind RIN-Werte aus 5 verschiedenen Blutproben gezeigt. Hierbei erfolgte die Isolierung von RNA aus 400 µL Vollblutproben durch direkte Lyse (d.h. erfindungsgemäß) und selektive Erythrozytenlyse. Direkte Lyse: Durchführung gemäß Ausführungsbeispiel 1. Selektive Erythrozytenlyse: Durchführung mit Qiagen, QIAamp RNA Blood Mini Kit, gemäß Handbuch. Darstellung der RNA-Integrität (RIN-Werte) nach Bioanalyzer-Analyse. Blut 1-5: Frischblut, Einzelspender 1-5, EDTA stabilisiert. Darstellung der Einzel- und Mittelwerte.

Die mit der direkten Lyse erzielten Werte von 7,8 im Mittel sind ausgezeichnet, zeigen eine hohe Integrität der RNA und belegen eindrucksvoll, dass RNasen während der Lyse und des Zellaufschlusses effizient inaktiviert werden. Zum Vergleich sind die RIN Werte nach selektiver Erythrozytenlyse gezeigt. Dies liegen mit einem Mittel von 6, 8 unter den Werten der direkten Lyse.

### Beispiel 2:

### Automation des Verfahrens zur RNA Isolierung aus Vollblut-Prozessierung unter Vakuum und in der Zentrifuge

Im Folgenden ist ein Verfahrensablauf für das Prozessieren von bis zu 96 Proben dargestellt. Die Lyse der Blutproben und die RNA Isolierung finden in 96well Platten statt. Auch hier wird die RNA nach der Lyse mit einem kommerziell verfügbaren RNA Reinigungskit der Fa. MACHEREY-NAGEL, NucleoSpin 8 RNA (REF 740698) oder NucleoSpin 96 RNA (740709) isoliert. Diese Kits enthalten die folgenden Bestandteile:
Lysis Buffer RA1 (hier nicht notwendig)
Wash Buffer RA2
Wash Buffer RA3 (concentrate)
Wash Buffer RA4 (concentrate)
DNase Reaction Buffer
rDNase, lyophilized
RNase-free water
NucleoSpin RNA Binding Strips (bei REF 740698) oder
NucleoSpin RNA Binding Plate (bei REF740709)
Wash Plate
Square Well Block
Elution Plate

Wash Buffer RA2 enthält GuSCN sowie Alkohol, Wash Buffer RA3 (wie oben) und RA4 70% Ethanol, Rest Wasser.

| **Schritt** | |
|---|---|
| 1 | 400 µL Vollblut vorlegen |
| 2 | Zugabe von 400 µL RNA Blood Lysis Buffer |
| 3 | Mix (Schüttler oder auf- und abpipettieren) |
| 4 | 10 µL Proteinase K (ca. 20 mg/mL) zugeben |
| 5 | mix (Schüttler, 5 s) |
| 6 | 15 min bei Raumtemperatur inkubieren, bevorzugt auf Schüttler |
| 7 | Zugabe von 400 µL 70% Ethanol. |
| 8 | Mix (Schüttler oder auf- und abpipettieren) |
| 9 | Das Lysat auf die NucleoSpin RNA Bindeplatten oder 8er Streifen auftragen |
| 10 | Ab hier dem Protokoll NucleoSpin 8/96 RNA folgen. Die Prozessierung kann entweder in einer Zentrifuge oder unter Vakuum erfolgen. |

Die Extraktion im 96well-Format ist beispielhaft in **Fig. 6** gezeigt. Hier wurden 8 Blutproben jeweils in 3-fach Bestimmung unter Zentrifugation und unter Vakuum prozessiert. Die Bestimmung der RNA-Menge erfolgte spektrophotometrisch bei 260 nm. Proben 1-8: Frischblut 400 µL, Einzelspender 1-8, n=3 (A, B, C), EDTA stabilisiert. Darstellung der Einzel- und Mittelwerte.

Diese Ergebnisse zeigen, dass beide Möglichkeiten zur Probenverarbeitung möglich sind. Die Schwankungen zwischen den einzelnen Blutproben repräsentiert wieder die Schwankungen im Hämatokrit (unterschiedliche Mengen an Leukozyten und damit unterschiedliche RNA Ausbeuten).

### Beispiel 3:

### Automation des Verfahrens zur RNA Isolierung aus Vollblut-Prozessierung unter Verwendung magnetischer Beads

Als feste Phase zur Bindung der isolierten RNA können auch andere als die zuvor beschriebenen Silica-Membranen eingesetzt werden. Im folgenden Ausführungsbeispiel werden anstelle der Membranen magnetische Beads eingesetzt. Die Separation und Isolierung der RNA erfolgt hierbei nicht durch Zentrifugation oder Vakuum sondern über magnetische Separation auf einem geeigneten Separator, z.B. mit statischen Magneten wie dem MACHEREY-NAGEL NucleoMag SEP, REF 744900, für 96 well-Platten.

Im Folgenden ist ein Verfahrensablauf für das Prozessieren von bis zu 96 Proben dargestellt. Die Lyse der Blutproben und die RNA Isolierung finden in 96well Platten statt. Hier wird die RNA nach der Lyse mit einem kommerziell verfügbaren RNA Reinigungskit der Fa. MACHEREY-NAGEL, NucleoMag 96 RNA (REF 744350) isoliert. Der Kit enthält folgende Bestandteile:
Lysis Buffer MR1 (hier nicht verwendet)
Binding Buffer MR2
Wash Buffer MR3
Wash Buffer MR4
Elution Buffer MR5
Magnetic beads
RNase free water
Reaction buffer for rDNase
rDNase (lyophilized)
Reducing Agent TCEP (hier nicht verwendet).

Der Bindepuffer MR2 enthält >90% Isopropanol, Rest Wasser. Wash Buffer MR3 enthält GuSCN sowie Alkohol, Wash Buffer MR4 ca. 80% Ethanol, Rest Wasser.

| **Schritt** | |
|---|---|
| 1 | 400 µL Vollblut vorlegen |
| 2 | Zugabe von 400 µL RNA Blood Lysis Buffer |
| 3 | Mix (Schüttler oder auf- und abpipettieren) |
| 4 | 10 µL Proteinase K (ca. 20 mg/mL) zugeben |
| 5 | mix (Schüttler, 5 s) |
| 6 | 15 min bei Raumtemperatur inkubieren, bevorzugt auf Schüttler |
| 7 | Zugabe von 28 µL magnetischer Beads und 400 µL Bindepuffer MR2. |
| 8 | Mix (Schüttler oder auf- und abpipettieren) |
| 9 | Ab hier dem Protokoll NucleoMag RNA folgen. |

Auch mit diesem Verfahren wurde eine Vielzahl von Blutproben extrahiert. Bei Verwendung von 400 µl Vollblut wurden je nach Probe 1-2 µg RNA mit einem Ratio A260/280 von etwa 1,6 isoliert. Das erfindungsgemäße Verfahren eignet sich also auch in Kombination mit magnetischer Separation der RNA.

### Beispiel 4:

### RNA Extraktion aus frischem und gefrorenem humanem Vollblut

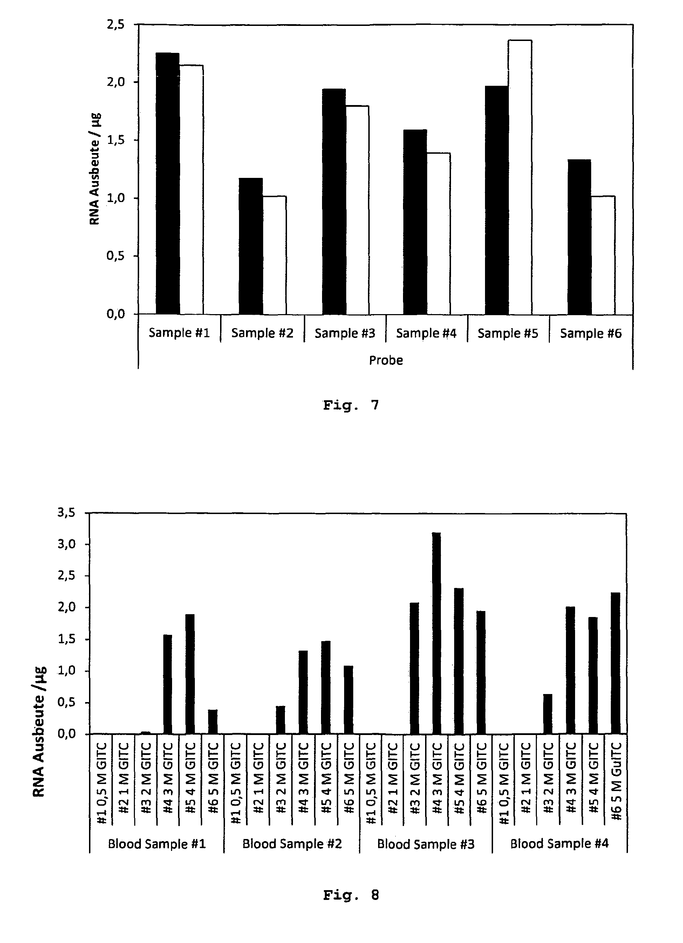

In diesem Versuch wurde das erfindungsgemäße Verfahren mit gefrorenen Blutproben durchgeführt. Hierzu wurden 6 frische EDTA Vollblutproben geteilt: Ein Teil der Proben wurde bei -20°C eingefroren, der andere Teil zum Vergleich über einen Zeitraum von 8h bei 4°C gelagert. Anschließend wurden die gefrorenen Proben aufgetaut und in einer parallelen Extraktion die RNA gemäß Ausführungsbeispiel 2 isoliert. Die RNA Ausbeute ist in **Fig. 7** dargestellt. Die Prozessierung erfolgte unter Vakuum gemäß Ausführungsbeispiel 2, die Bestimmung der RNA-Menge spektrophotometrisch bei 260 nm. Schwarz: frische Blutproben, Weiß: gefrorene Blutproben. Proben 1-6: Frischblut 200 µL, Einzelspender 1-6, EDTA stabilisiert. Darstellung der Mittelwerte von n=3.

Die Ergebnisse belegen, dass das erfindungsgemäße Verfahren gleichermaßen für frische, wie gefrorene Proben angewendet werden kann. Ebenfalls untersucht wurde die RNA Reinheit (A260/280), die RNA Qualität (RIN) sowie die Amplifizierbarkeit mittels RT-PCR untersucht. Auch hier gab es keine signifikanten Unterschiede zwischen den frischen und gefrorenen Proben: die RNA Reinheit und Qualität war vergleichbar und auch bei der Amplifizierbarkeit wurden keine Unterschiede beobachtet.

### Beispiel 5:

### Isolierung von RNA aus Tierblut

Exemplarisch für die Verwendung von Tierblut wurde an dieser Stelle RNA aus gefrorenem Pferdeblut gemäß Ausführungsbeispiel 2 isoliert. Aus insgesamt 5 Einzelblutproben konnte in jedem Fall RNA isoliert werden. In keinem Fall kam es zu einem Verstopfen der Bindeplatte oder anderen Auffälligkeiten, alle Proben konnten problemlos unter Vakuum prozessiert werden. Somit konnte gezeigt werden, dass das erfindungsgemäße Verfahren nicht nur für humanes Vollblut, sondern auch für Blutproben anderer Spezies geeignet ist.

### Beispiel 6:

### Lysepufferkonzentrationen und Komponenten

Der erfindungsgemäße Lysepuffer muss sicherstellen, dass nach Zugabe der Blutprobe mehrere Aufgaben gelöst werden. So müssen die vier folgenden Voraussetzungen erfüllt sein:
1. Die Proteinase darf durch den Puffer nicht inaktiviert werden. Es muss sichergestellt sein, dass auch unter den gewählten Hochsalzbedingungen noch ein effizienter Proteinverdau stattfindet.
2.RNasen müssen inaktiviert werden um die Qualität der isolierten RNA nicht negativ zu beeinflussen.
3. Die Bindung der RNA an eine feste Phase muss unter den gewählten Bedingungen im Zusammenspiel mit Ethanol möglich sein.
4.Die Lyse des Vollblutes muss sichergestellt sein.

Besonders relevant ist in diesem Kontext die Konzentration des chaotropen Salzes. Auf der einen Seite führt das Salz zur Lyse der Blutzellen (Merkmal 4), es inaktiviert die RNasen (Merkmal 2) und es führt zu Bindung der RNA an die feste Phase (Merkmal 3). Ist die Konzentration hingegen zu hoch, so werden Proteinasen inaktiviert und die Lyse ist unvollständig; ein Verstopfen der Säule kann unter diesen Bedingungen nicht verhindert werden (Merkmal 1).

Es gilt also, die Bedingungen so zu wählen, dass ein optimales Zusammenspiel aus Chaotropsalz und Proteinase erzielt wird. In einem ersten Experiment wurde die Konzentration an GuSCN im Lysepuffer gemäß Ausführungsbeispiel 2 im Konzentrationsbereich von 0.5, 1, 2, 3, 4 und 5 M variiert. Prozessiert wurden 4 individuelle Blutproben.

Als Ergebnis wurde festgestellt, dass die Wells bei 0,5 und 1 M GuSCN verstopften. Trotz Anwesenheit von Proteinase in Ansatz reicht die Lyseeffizienz des Lysepuffers (Endkonzentration GuSCN 0,25 und 0, 5 M) nicht aus, um die Blutproben vollständig aufzuschließen. Proteinase allein führt also nicht zu einer zufriedenstellenden Lyse der Blutproben.

Als nächstes wurde die Bindung von RNA an die feste Phase untersucht. Als Parameter dient hier die RNA Ausbeute. In **Fig. 8** sind die Ergebnisse der Untersuchung des Einflusses der Konzentration des Chaotropsalzes auf die RNA Ausbeute zusammengefasst. Die Isolierung der RNA erfolgte durch direkte Lyse aus Vollblutproben im 96well Format mit NucleoSpin 96 RNA. Die Prozessierung wurde unter Vakuum vollzogen und die RNA-Menge spektrophotometrisch bei 260 nm bestimmt. Es wurden 4 Einzelblutproben eingesetzt. Darstellung der Mittelwerte von n=3.

Zunächst ist hier zu sehen, dass die GuSCN Konzentrationen von 0,5 und 1 M keine Ausbeuten lieferten, da die Wells verstopften und keine Isolierung durchgeführt werden konnte. Eine Konzentration von 2 M GuSCN führt bereits zur RNA Isolierung, wobei das Verfahren ab Konzentrationen von 3 M optimal abläuft, die RNA Ausbeuten sind hier höher als bei 2 M GuSCN.

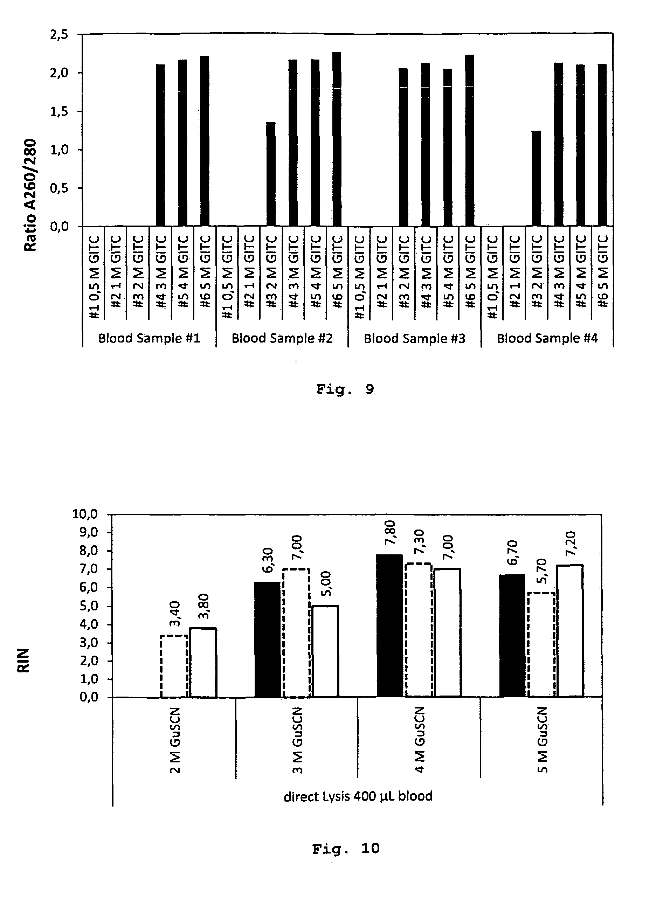

Die RNA Reinheit in Abhängigkeit von der Chaotropsalzkonzentration ist in **Fig. 9** dargestellt. Die Isolierung von RNA aus Vollblutproben erfolgte durch direkte Lyse im 96well Format mit NucleoSpin 96 RNA. Die Prozessierung wurde unter Vakuum vollzogen und die RNA-Menge über spektrophotometrische Bestimmung des A260/280 Ratios bestimmt. Es wurden 4 Einzelblutproben eingesetzt. Darstellung der Mittelwerte von n=3.

Auch hier wurden für GuSN Konzentrationen ab 2 M optimale Ergebnisse mit Ratios von ca. 2,1 ermittelt. Grundsätzlich funktioniert das Verfahren auch mit 1,5 M GuSCN, die Ratios sind aber etwas niedriger und auch die Schwankungen über die Blutproben sind etwas größer.

Die RNA Qualität in Abhängigkeit von der Chaotropsalzkonzentration ist in **Fig. 10** dargestellt.

Die Isolierung von RNA aus Vollblutproben erfolgte durch direkte Lyse im 96well Format mit NucleoSpin 96 RNA. Die Prozessierung wurde unter Vakuum und die Bestimmung der RNA-Integrität (RIN) mittels Bioanalyzer Messungen durchgeführt. Es wurden Proben von 3 Einzelblutproben (gekennzeichnet durch die Farben Schwarz, Grau und Weiß) analysiert. Die Probe 1 (Schwarz) bei 2 M GuSCN verstopfte im Bioanalyzer Lauf und lieferte keine RIN. Darstellung der Einzelwerte.

Hohe RIN-Werte und damit hohe RNA-Qualität und Integrität wurden für GuSCN Konzentrationen > 2 M erhalten. Grundsätzlich funktioniert das Verfahren auch mit 1,5 oder 2 M GuSCN, die RIN Werte sind aber etwas niedriger als bei höheren Konzentrationen, dennoch werden auch hier bereits RNasen offensichtlich zumindest teilweise durch das Chaotropsalz inaktiviert. Ist die Molarität des Lysepuffers bei 3 M oder höher, so ist die Inaktivierung noch effizienter, was an erhöhter RNA Integrität und erhöhten RIN Werten erkennbar ist.

Der Effekt der Chaotropsalzkonzentration auf die Aktivität der Proteinase wurde in einem separaten Experiment untersucht. Grundsätzlich wurden die zuvor gezeigten Experimente gemäß Ausführungsbeispiel 2 wiederholt, jedoch in An- bzw. Abwesenheit der Proteinase K. Die Ergebnisse sind in der folgenden Tabelle zusammengefasst:
Als einheitliche Probe für alle Ansätze wurde ein Master-Blutpool eingesetzt. Dargestellt sind Mittelwerte von n=3.

| | Ohne Proteinase | | | | | | Mit Proteinase | | | | | |
|---|---|---|---|---|---|---|---|---|---|---|---|---|
| Konz. GuSCN | 0,5 | 1 | 2 | 3 | 4 | 5 | 0,5 | 1 | 2 | 3 | 4 | 5 |
| Verstopfung | Ja | Ja | Ja | Ja | Ja | Nein | Ja | Ja | Nein | Nein | Nein | Nein |
| RNA Ausbeute µg | -/- | -/- | -/- | -/- | -/- | 0,39 | -/- | -/- | 1,2 | 2,6 | 2,6 | 2,6 |
| RNA Reinheit A260/280 | -/- | -/- | -/- | -/- | -/- | 1,4 | -/- | -/- | 1,82 | 1,97 | 1,97 | 2,0 |
| RIN | -/- | -/- | -/- | -/- | -/- | -/- | -/- | -/- | 3,3 | 6,8 | 6,9 | 7,4 |

Aus dieser Tabelle wird ersichtlich, dass es ohne Proteinasezugabe zu einer unvollständigen Lyse kommt und die Bindeplatte verstopft. Erst mit Chaotropsalzkonzentrationen von 5 M war die Lyse so effizient, dass keine Verstopfung beobachtet wurde. Die RNA Ausbeute und Reinheit war jedoch gering.

Zudem wurde beobachtet, dass die Silicamembran nach Lyse und Waschen immer noch deutlich dunkel gefärbt war (Kontaminationen von Zellresten und Proteinen), während die Membranen nach Lyse mit Proteinase K weiß und frei von Rückständen waren.

In Gegenwart von Proteinase führen Chaotropsalzkonzentrationen von > 1 M zu einer vollständigen Lyse (keine Verstopfung der Membran). Die RNA Ausbeute steigt von 2 zu 3 M, bleibt dann relativ konstant. Ebenso verhält es sich mit der RNA Reinheit. Diese ist bei 2 M gut, bessere Werte werden ab 3 M GuSCN erreicht. Die RNA Qualität, gemessen an der RIN, ist ebenfalls bei 2 M GuSCN akzeptabel, ab 3 M sehr gut.

Als Fazit kann festgehalten werden, dass selbst GuSCN Konzentrationen von 4 und 5 M eine ausreichend hohe Proteinaseaktivität sicherstellen. Fehlt die Proteinase, ist die Lyse ineffizient und unvollständig.

### Beispiel 7:

### Einfluss von MgCl₂ und anderen zweiwertige Metallionen auf die Lyseeffizienz und die RNA Ausbeute

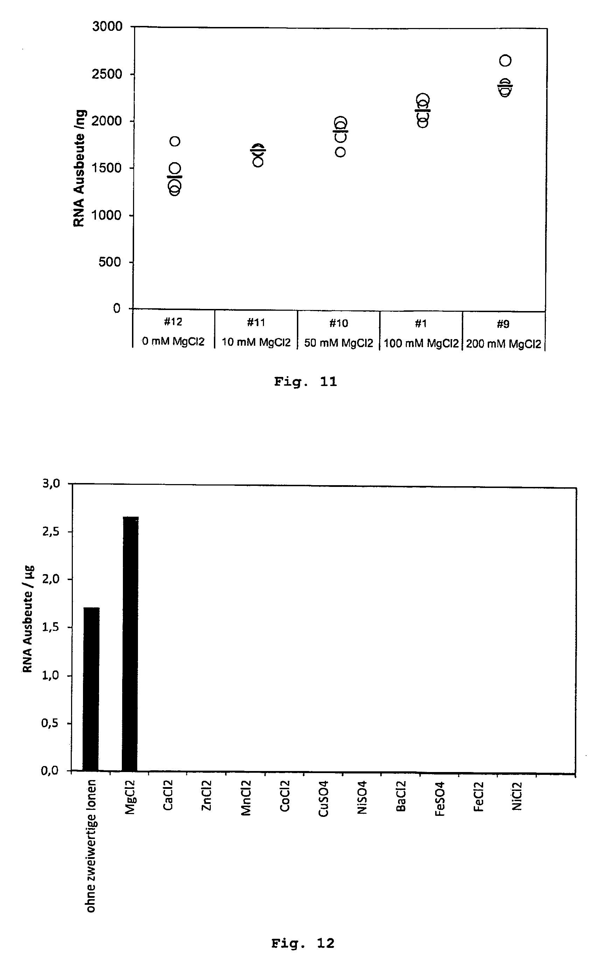

In einem ersten Versuch wurde die Konzentration des Magnesiumchlorids im Lysepuffer variiert. Die Durchführung erfolgte gemäß Ausführungsbeispiel 1, die Ergebnisse sind in **Fig. 11** zusammengefasst. Die Isolierung der RNA erfolgte aus Vollblut durch direkte Lyse gemäß Ausführungsbeispiel 1. Bestimmung der RNA-Ausbeute durch RiboGreen Quantifizierung. Es wurde ein Blutpool eingesetzt, jeweils n=4. Darstellung der Einzelwerte und Mediane.

Die Ergebnisse belegen, dass durch den Zusatz von MgCl₂ eine weitere Steigerung der RNA Ausbeute möglich ist und sich durch die Zugabe von bis zu 200 mM MgCl₂ im Lysepuffer nahezu verdoppeln lässt. Unabhängig von der Konzentration der Magnesiumionen konnten die Proben ohne Probleme prozessiert werden, ein Verstopfen der Membran wurde nicht beobachtet.

In einem weiteren Versuch gemäß Ausführungsbeispiel 2 wurde die Menge an MgCl₂ noch weiter erhöht. Dabei wurden folgende Ergebnisse erzielt:

| **Eingesetzte Menge MgCl2** | **RNA Ausbeute** |
|---|---|
| 0 mM | 1,22 µg |
| 100 mM | 2,28 µg |
| 200 mM | 2,36 µg |
| 400 mM | 2,24 µg |
| 800 mM | 2,04 µg |

Dies belegt, dass eine zunehmende Menge an Magnesiumionen zu einer Erhöhung der RNA Ausbeute führt. Das Optimum liegt im vorliegenden Fall bei ca. 200 mM; wird die Konzentration weiter erhöht, so funktioniert das erfindungsgemäße Verfahren weiterhin, die RNA Ausbeute geht jedoch gegenüber dem Optimum wieder leicht zurück.

Dieser Effekt durch die Verwendung von MgCl₂ ist auch insofern überraschend, als dass andere zweiwertige Ionen (mit Ausnahme von Calcium unter bestimmten Umständen) im gleichen Konzentrationsbereich zu einer kompletten Verstopfung der Membran führen. Die Ionen bedingen eine Präzipitation, so dass die Proben nicht weiter prozessiert werden können. Die RNA Ausbeuten sind entsprechend niedrig oder null. Die RNA Ausbeute in Abhängigkeit von den eingesetzten Ionen ist in **Fig. 12** dargestellt. Die Isolierung der RNA erfolgte aus Vollblut durch direkte Lyse gemäß Ausführungsbeispiel 2. Alle Metallionen wurden mit einer Konzentration von 100 mM im Lysepuffer eingesetzt. Bestimmung der RNA-Ausbeute durch RiboGreen Quantifizierung. Es wurde ein Blutpool eingesetzt, jeweils n=3, Darstellung der Mittelwerte. Mit Ausnahme der Ansätze ohne zweiwertige Ionen und mit MgC12 führten alle übrigen Kombinationen zu einem Verstopfen der Membran und damit Null-Ausbeuten an RNA.

In weiteren Experimenten wurde festgestellt, dass neben Magnesium- auch Calciumionen einen positiven Effekt auf die RNA-Ausbeute haben. Beim Einsatz von Calciumionen hängt dieser Effekt jedoch zusätzlich noch vom eingesetzten chaotropen Salz ab. So wurde beobachtet, dass Calciumionen (als CaCl₂) in Kombination mit Guanidiniumthiocyanat keinen fördernden Effekt haben und die Membranen verstopfen. In Kombination mit Alkalithiocyanat wie Natriumthiocyanat konnte allerdings eine Verbesserung der RNA-Ausbeute beobachtet werden. Die Calciumionenkonzentration bei der dieser vorteilhafte Effekt besonders ausgeprägt erscheint liegt bei etwa 50-100 mM.

Diese Versuche zeigen, dass das erfindungsgemäße Verfahren ganz bevorzugt mit Magnesiumionen, sowie auch mit Calciumionen funktioniert und sich dabei eine Verbesserung der RNA-Ausbeute einstellt. Andere zweiwertige Metallionen haben hingegen keinen positiven, sondern im Gegenteil, einen ausgesprochen negativen Effekt, da sie zur Ausfällung von Zellbestandteilen führen.

### Beispiel 8

### Einsatz verschiedener Detergenzien im Lysepuffer

Im Folgenden wurde der Einfluss der Detergenzien auf die Lyseeffizienz und RNA Ausbeute untersucht. Dabei wurden Detergenzien einzeln oder im Gemisch eingesetzt. Getestet wurden folgende Kombinationen:

| **Probennummer** | **Nicht-ionisches Detergenz** | **Anionisches Detergenz** |
|---|---|---|
| 1 | 50 g/L Brij58 | 0,15 g/L N-Lauroyl-Sarkosin |
| 2 | 50 g/L Brij58 | - |
| 3 | 50 g/L Brij58 | 0,5 g/L SDS |
| 4 | 50 g/L Brij58 | 0,5 g/L Docusat |
| 5 | 50 g/L Tween20 | 0,15 g/L N-Lauroyl-Sarkosin |
| 6 | 50 g/L Brij56 | 0,15 g/L N-Lauroyl-Sarkosin |
| 7 | 50 g/L Brij35 | 0,15 g/L N-Lauroyl-Sarkosin |
| 8 | 50 g/L Brij56 | 0,5 g/L SDS |

Das Chaotropsalz, sowie die Konzentration von MgCl₂ wurde über alle Ansätze konstant gehalten (3 M GuSCN, 100 mM MgCl₂; Durchführung gemäß Ausführungsbeispiel 2).

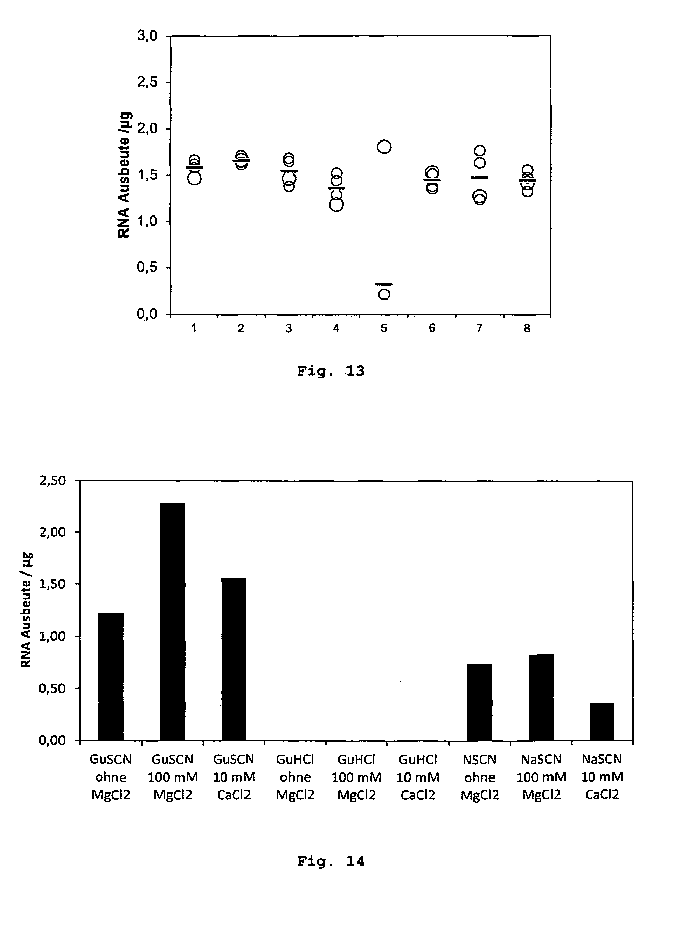

Die **Fig. 13** zeigt den Einfluss verschiedener Detergenzien und Detergenzkombinationen auf die RNA Ausbeute und die Lyseeffizienz. Isolierung von RNA aus Vollblut durch direkte Lyse gemäß Ausführungsbeispiel 2. Einzelwerte und Median von n=4.

Als Fazit kann man festhalten, dass sowohl einzelne Detergenzien, wie auch Kombinationen aus nicht-ionischen und anionischen Detergenzien gemäß dem erfindungsgemäßen Verfahren eingesetzt werden können. Lediglich die Kombination Nr. 5 mit Tween 20 führte zu größeren Ausbeuteschwankungen und im Mittel zu reduzierten RNA Ausbeuten. Eine Verstopfung der Membranen wurde dennoch nicht beobachtet, so dass das erfindungsgemäße Verfahren auch mit Tween 20 funktioniert.

Das Detergenz unterstützt die Lyse, löst Lipide der lysierten Blutzellen und verhindert auf diese Weise ein Verstopfen der Membranen. Wird hingegen komplett auf das Detergenz verzichtet, so kommt es zu einer unvollständigen Lyse und einem Verstopfen der Membranen.

### Beispiel 9

### Einsatz verschiedener Chaotropsalze im Lysepuffer

Um den Einfluss verschiedener Chaotropsalze zu untersuchen, wurden Guanidiniumthiocyanat (GuSCN), Guanidiniumhydrochlorid (GuHCl) und Natriumthiocyanat (NaSCN) als chaotrope Substanzen im Lysepuffer gemäß Ausführungsbeispiel 2 getestet, wobei das Chaotropsalz jeweils in einer Konzentration von 3 M eingesetzt wurde. Mittelwerte von n=3. Die Ergebnisse sind in **Fig. 14** dargestellt.

Für Guanidiniumthiocyanat erkennt man den klaren Einfluss von Magnesiumionen auf die RNA Ausbeute, ebenso den fördernden Effekt von Calciumionen. Bei Verwendung von Natriumthiocyanat ist die RNA-Ausbeute generell niedriger, dennoch funktioniert das Verfahren ohne zweiwertige Ionen, mit 10 mM CaCl₂ und mit 100 mM MgCl₂. Bei Verwendung von Guanidiniumhydrochlorid verstopften die Membranen.

Damit kann geschlussfolgert werden, dass sich chaotrope Salze auf Basis des Thiocyanations, ganz bevorzugt Guanidiniumthiocyanat, für das erfindungsgemäße Verfahren eignen.

## Patentansprüche

1. Verfahren zur Isolierung von RNA aus einer Vollblutprobe umfassend die Schritte:
a) Inkontaktbringen der Probe mit einer wässrigen Lyse-Lösung enthaltend wenigstens eine Lysesubstanz in einer Konzentration von 1,5 Mol/l bis 7 Mol/l wenigstens ein Detergenz und Magnesiumionen,
b) zeitgleiche oder anschließende Zugabe einer Proteinase, insbesondere Proteinase K,
c) danach Inkubieren der nach Schritt b) erhaltenen Lösung zum wenigstens teilweisen enzymatischen Verdau und Lyse der Probe wobei ein Lysat gewonnen wird und
d) Isolierung der RNA aus dem Lysat,
wobei die Probe im Schritt a) unmittelbar mit der Lyse-Lösung in Kontakt gebracht wird und bis zum Abschluss des Schritts c) nicht durch Zugabe eines weiteren Lösungsmittels verdünnt, kein Stabilisator zugegeben und/oder nicht zentrifugiert wird, wobei das Gesamtvolumen aus Probe, wässriger Lyse-Lösung und Proteinase bis zum Abschluss des Schritts c) das Ausgangsvolumen der Probe um nicht mehr als den Faktor 2,5 übersteigt, vorzugsweise um nicht mehr als den Faktor 1,5.

2. Verfahren nach Anspruch 1, **dadurch gekennzeichnet, dass** im Schritt c) in der Lösung enthaltene RNasen weitestgehend vollständig inaktiviert werden.

3. Verfahren nach Anspruch 1 oder 2, **dadurch gekennzeichnet, dass** die wässrige Lyse-Lösung die Lysesubstanz in einer Konzentration von 2 Mol/l bis 6 Mol/l enthält, insbesondere von 2, 5 Mol/l bis 5 Mol/l, bevorzugt von 3 Mol/l bis 4 Mol/l.

4. Verfahren nach einem der vorstehenden Ansprüche, **dadurch gekennzeichnet, dass** die wässrige Lyse-Lösung mit der Probe in einem Volumenverhältnis von 1,8 : 1 bis 1 : 1,8 vermischt wird, insbesondere von 1,5 : 1 bis 1 : 1,5, vorzugsweise von 1,2 : 1 bis 1 : 1,2.

5. Verfahren nach einem der vorstehenden Ansprüche, **dadurch gekennzeichnet, dass** im Schritt b) die Proteinase als Feststoff zusammen mit einem Lösungsmittel oder als Proteinase-Lösung zugesetzt wird, wobei die jeweilige Flüssigkeitsmenge so eingestellt wird, dass die Flüssigkeitsmenge höchstens 20 % des Volumens aus Probe und wässriger Lyse-Lösung beträgt, insbesondere höchstens 10 %.

6. Verfahren nach einem der vorstehenden Ansprüche, **dadurch gekennzeichnet, dass** die Lysesubstanz ein oder mehrere Chaotropsalze umfasst, und insbesondere ausgewählt ist aus Thiocyanaten, wie Natriumthiocyanat, Kaliumthiocyanat und Guadiniumthiocyanat, Harnstoff, Perchloratsalzen wie Natriumperchlorat und/oder Kaliumiodid.

7. Verfahren nach einem der vorstehenden Ansprüche, **dadurch gekennzeichnet, dass** die Lyse-Lösung Magnesiumionen in einer Konzentration von 10 bis 1000 mMol/l, bevorzugt von 100 bis 600 mMol/l, weiter bevorzugt 150 bis 400 mMol/l enthält.

8. Verfahren nach einem der vorstehenden Ansprüche, **dadurch gekennzeichnet, dass** die Lyse-Substanz Kalium- und/oder Natriumthiocynat umfasst und die Lyse-Lösung Calciumionen enthält, wobei die Konzentration an Calciumionen insbesondere 10 bis 150 mMol/l beträgt, bevorzugt von 50 bis 100 mMol/l.

9. Verfahren nach einem der vorstehenden Ansprüche, **dadurch gekennzeichnet, dass** die Lyse-Lösung frei von zwei- oder mehrwertigen Metallionen mit Ausnahme von Magnesium und Calcium ist.

10. Verfahren nach einem der vorstehenden Ansprüche, **dadurch gekennzeichnet, dass** das Detergenz ausgewählt ist aus nicht-ionischen Detergenzien, anionischen Detergenzien oder Mischungen von diesen, insbesondere Polyoxyethylen-20-Cetylether (Brij 58®) oder N-Lauroyl-Sarkosin.

11. Verfahren nach einem der vorstehenden Ansprüche, **dadurch gekennzeichnet, dass** die Lyse-Lösung weitere Inhaltsstoffe aufweist, die insbesondere ausgewählt sind aus Puffersubstanzen, Enzymen, Reduktionsmittel und/oder Alkoholen.

12. Verfahren nach einem der vorstehenden Ansprüche, **dadurch gekennzeichnet, dass** zur Isolierung der RNA aus dem Lysat die RNA an eine feste Phase angelagert wird, gewünschtenfalls gefolgt von einem oder mehreren Waschschritten.

13. Verfahren nach Anspruch 12, **dadurch gekennzeichnet, dass** zur Anlagerung der RNA an die feste Phase dem Lysat ein Bindungsmittel zugegeben wird, insbesondere ein Alkohol, vorzugsweise Ethanol und/oder Isopropanol, sowie eine Alkohol/Wasser Mischung.

14. Verfahren nach einem der vorstehenden Ansprüche, **dadurch gekennzeichnet, dass** die DNA zumindest teilweise, bevorzugt nahezu vollständig entfernt wird, insbesondere durch einen DNase-Verdau.

15. Verfahren nach einem der vorstehenden Ansprüche, **dadurch gekennzeichnet, dass** die isolierte RNA gewonnen wird, insbesondere durch Desorption.

16. Verwendung eines Kits zur Isolierung von RNA aus einer Vollblutprobe nach einem der Ansprüche 1 bis 15, enthaltend zumindest die Bestandteile:
a) eine wässrige Lyse-Lösung enthaltend wenigstens eine Lysesubstanz in einer Konzentration von 1 Mol/l bis 5 Mol/l
b) wenigstens ein Detergenz,
c) Magnesiumionen,
d) eine Proteinase, insbesondere Proteinase K.

## Claims

1. A method for isolating RNA from a whole blood sample comprising the steps:
a) bringing the sample into contact with an aqueous lysis solution containing at least one lysis substance at a concentration of 1.5 mol/l to 7 mol/l and at least one detergent, and magnesium ions,
b) simultaneous or subsequent addition of a proteinase, in particular proteinase K,
c) then incubating the solution obtained according to step b) for at least partial enzymatic digestion and lysis of the sample, a lysate being obtained, and
d) isolation of the RNA from the lysate
wherein the sample is brought directly in contact with the lysis solution in step a) and is not diluted by adding a further solvent until the completion of step c), no stabiliser is added and/or it is not centrifuged, in particular the overall volume comprising the sample, the aqueous lysis solution and the proteinase exceeding the initial volume of the sample by no more than the factor 2.5, preferably by no more than the factor 1.5, by the completion of step c).

2. The method according to claim 1, **characterized in that** RNases contained in the solution in step c) are as far as possible totally inactivated.

3. The method according to claim 1 or 2, **characterised in that** the aqueous lysis solution contains the lysis substance at a concentration of 2 mol/l to 6 mol/l, in particular of 2.5 mol/l to 5 mol/l, preferably of 3 mol/l to 4mol/l.

4. The method according to any of the preceding claims, **characterised in that** the aqueous lysis solution is mixed with the sample at a volume ratio of 1.8 : 1 to 1 : 1.8, in particular of 1.5 : 1 to 1 : 1.5, preferably of 1.2 : 1 to 1 : 1.2.

5. The method according to any of the preceding claims, **characterised in that** in step b) the proteinase is added as a solid together with a solvent or as a proteinase solution, the respective quantity of liquid being set such that the quantity of liquid ist at most 20% of the volume comprising the sample and the aqueous lysis solution, in particular at most 10%.

6. The method according to any of the preceding claims, **characterised in that** the lysis substance comprises one or more chaotropic salts, and in particular is selected from thiocyanates such as sodium thiocyanate, sodium thiocyanate and guanidinium thiocyanate, urea, perchlorate salts such as sodium perchlorate and/or potassium iodide.

7. The method according to any of the preceding claims, **characterised in that** the lysis solution contains magnesium ions, in particular at a concentration of 10 to 1000 mMol/l, preferably of 100 to 600 mM/l, more preferably 150 to 400 mMol/l.

8. The method according to any of the above claims, **characterised in that** the lysis substance includes potassium and/or sodium thiocyanete and the lysis solution contains calcium ions, the calcium ion concentration being in particular 10 to 150 mMol/l, preferably 100 mMol/l.

9. The method according to any of the above claims, **characterised in that** the lysis solution is free from bivalent of multivalent metal ions with the exception of magnesium and calcium.

10. The method according to any of the above claims, **characterised in that** the detergent is selected from non-ionic detergents, anionic detergents or mixtures of the latter, in particular polyoxyethylene-20-cetyl ether (Brij 58®) or N-lauroyl-sarcosine.

11. The method according to any of the preceding claims, **characterised in that** the lysis solution has further ingredients which are chosen in particular from buffer substances, enzymes, reduction agents and/or alcohols.

12. The method according of any of the preceding claims, **characterised in that** in order to isolate the RNA from the lysate the RNA is attached to a solid phase, desirably followed by one or more washing steps.

13. The method according to claim 12, **characterised in that** for attachment of the RNA to the solid phase a binding agent is added to the lysate, in particular an alcohol, preferably ethanol and/or isopropanol, and an alcohol/water mixture.

14. The method according to any of the preceding claims, **characterised in that** the DNA is at least partially, preferably almost totally removed, in particular by DNase digestion.

15. The method according to any of the preceding claims, **characterised in that** the isolated RNA is extracted, in particular by desorption.

16. Use of a kit for isolating RNA from a whole blood sample, containing at least the components:
a) an aqueous lysis solution containing at least one lysis substance at a concentration of 1 mol/l to 5 mol/l,
b) at least one detergent,
c) magnesium ions,
d) a proteinase, in particular proteinase K.

## Revendications

1. Procédé d'extraction de l'ARN d'un échantillon de sang total comprenant les étapes suivantes :
a) Mise en contact de l'échantillon avec une solution de lyse aqueuse contenant au moins une substance de lyse en une concentration comprise entre 1,5 Mol/l et 7 Mol/l, au moins un détergent et des ions de magnésium,
b) l'ajout simultané ou consécutif d'une protéinase, plus spécifiquement d'une protéinase K,
c) ensuite, l'incubation de la solution obtenue en b) au moins jusqu'à la digestion enzymatique partielle et la lyse de l'échantillon, ce qui permet d'obtenir un lysat ; puis
d) l'isolation de l'ARN contenu dans le lysat,
l'échantillon en a) étant alors immédiatement mis en contact avec la solution de lyse et n'étant pas dilué par l'ajout d'un autre solvant avant la fin de l'étape c), aucun stabilisateur n'étant ajouté ou centrifugé, et le volume total de l'échantillon, de la solution de lyse aqueuse et de la protéinase ne dépassant jusqu'à la fin de l'étape c) le volume de départ de l'échantillon que par un facteur maximal de 2,5, idéalement pallefactor de 1,5.

2. Procédé selon la revendication 1, **caractérisé en ce que** les ARNases contenus dans la solution à l'étape C) sont complètement désactivés dans la plus grande proportion possible.

3. Procédé selon les revendications 1 ou 2, **caractérisé en ce que** la solution de lyse aqueuse contient la substance de lyse dans une concentration comprise entre 2 Mol/l et 6 Mol/l, plus précisément de 2,5 Mol/l à 5 Mol/l, idéalement de 3 Mol/l à 4 Mol/l.

4. Procédé selon l'une des revendications précédentes, **caractérisé en ce que** la solution de lyse aqueuse est mélangée avec l'échantillon selon un rapport volumique de 1,8 : 1 à 1 : 1,8, plus précisément de 1,5 : 1 à 1 : 1,5, idéalement de 1,2 : 1 à 1 : 1,2.

5. Procédé selon l'une des revendications précédentes, **caractérisé en ce que** la protéinase à l'étape b) est ajoutée en tant que matière solide conjointement avec un solvant ou comme solution de protéinase, la quantité de fluide respective étant jaugée de façon à ce que la quantité de fluide compose au maximum 20 % du volume de l'échantillon et de la solution de lyse aqueuse, idéalement 10 %.

6. Procédé selon l'une des revendications précédentes, **caractérisé en ce que** la substance de lyse comprend un ou plusieurs sels chaotropiques et est notamment choisie parmi des thiocyanates, comme le cyanate de sodium, le cyanate de potassium et le thiocyanate de guanidinium, l'urée, les sels de perchlorates comme le perchlorate de sodium ou l'iodure de potassium.

7. Procédé selon l'une des revendications précédentes, **caractérisé en ce que** la solution de lyse contient des ions de magnésium dans une concentration comprise entre 10 et 1000 mMol/l, préférablement entre 100 et 600 mMol/l, idéalement entre 150 et 400 mMol/l.

8. Procédé selon l'une des revendications précédentes, **caractérisé en ce que** la substance de lyse comprend un cyanate de potassium et/ou de sodium et que la solution de lise contient des ions de calcium, la concentration en ions de calcium étant notamment comprise entre 10 et 150 mMol/l, idéalement entre 50 et 100 mMol/l.

9. Procédé selon l'une des revendications précédentes, **caractérisé en ce que** la substance de lyse est exempte d'ions métalliques di- ou multivalents, à l'exception du magnésium et du calcium.

10. Procédé selon l'une des revendications précédentes, **caractérisé en ce que** le détergent est sélectionné parmi des détergents non ioniques, anioniques ou des mélanges de ces derniers, notamment le polyoxypropylène (20) cétyléther (Brij 58®) ou la sarcosine N-lauroyl.

11. Procédé selon l'une des revendications précédentes, **caractérisé en ce que** la solution de lyse présente d'autres composants qui sont spécialement sélectionnés parmi des substances tampons, des enzymes, des agents réducteur et/ou alcools.

12. Procédé selon l'une des revendications précédentes, **caractérisé en ce que** aux fins de l'isolation de l'ARN contenu dans le lysat, l'ARN est attaché à une phase solide puis soumis au besoin à une ou plusieurs étapes de nettoyage.

13. Procédé selon la revendication 12, **caractérisé en ce qu'**est ajouté un liant au lysat aux fins la liaison de l'ARN à la phase solide, notamment un alcool, préférablement de l'éthanol et/ou isopropanol, ainsi qu'un mélange d'eau/alcool.

14. Procédé selon l'une des revendications précédentes, **caractérisé en ce que** l'ADN est retiré au moins partiellement et idéalement presque complètement, notamment au moyen de la digestion par la DNase.

15. Procédé selon l'une des revendications précédentes, **caractérisé en ce que** l'ARN isolé est obtenu, notamment par désorption.

16. Utilisation d'une trousse d'isolation de l'ARN à partir d'un échantillon de sang total selon l'une des revendications 1 à 15 comprenant au moins les composants suivants :
a) une solution de lyse aqueuse contenant au moins une substance de lyse en une concentration comprise entre 1 Mol/l et 5 Mol/l
b) au moins un détergent,
c) des ions de magnésium,
d) une protéinase, plus spécifiquement une protéinase K.
